# EUROPEAN PATENT SPECIFICATION

(11) **EP 2 730 584 B1**
(45) Date of publication and mention of the grant of the patent: **18.04.2018**
(21) Application number: 12793167.3
(22) Date of filing: 28.02.2012
(51) Int. Cl.: A61K 35/30, A61K 35/54, A61K 38/02

(54) **PROTEIN-POLYPEPTIDE COMPLEX OBTAINED FROM NERVOUS TISSUE OF HOOFED LIVESTOCK EMBRYOS, PROCESS FOR PREPARING SAME AND PHARMACEUTICAL COMPOSITION**
PROTEINPOLYPEPTIDKOMPLEX AUS NERVENGEWEBE VON HUFTIEREMBRYONEN, VERFAHREN ZU SEINER HERSTELLUNG UND PHARMAZEUTISCHE ZUSAMMENSETZUNG DARAUS
COMPLEXE DE PROTÉINE-POLYPEPTIDE PROVENANT DE TISSU NERVEUX D'EMBRYONS D'ONGULÉS, PROCÉDÉ DE PRODUCTION ET COMPOSITION PHARMACEUTIQUE LE COMPRENANT

(30) Priority: 30.05.2011 RU 2011121686
(43) Date of publication of application: 14.05.2014
(73) Proprietor: Aktsionernoe Obschestvo "Pharm-Sintez", Moskow, 111024 (RU)
(72) Inventor: NAZARENKO, Anna Borisovna, Moscow 121614 (RU); SOKOLOV, Mikhail Anatolevich, Moscow 107150 (RU)
(74) Representative: Held, Stephan
(86) International application number: PCT/RU2012/000136
(87) International publication number: WO 2012/166004

(56) References cited:
- WO-A1-2009/088314
- WO-A1-2010/007620
- EA-A- 200 000 634
- EA-B1- 003 301
- RU-C1- 2 128 511
- SPRAVOCHNIK VIDAL.: 'Lekarstvennye preparaty v Rossii. Spravochnik' IZDANIE XVI. M. ASTRAFARMSERVIS 2010, pages 506 - 507

## Description

### Field

The invention relates to a biologically active substance - a protein/polypeptide complex (PPC), prepared from fetal nerve tissue of of hoofed farm animals, making antihypoxic, vascular, and neuroprotective effect, stimulating physiological and reparative regeneration of nerve tissue, intended for use in medicine production for central and peripheral nervous system diseases, hypoxic states, in disaster and sports medicine, the method for production of the same and a pharmcomposition based on it.
PPC - protein/polypeptide complex;
PPNC - protein/polypeptide/nucleotide complex;
I - ionic strength of solution;
Pharmcomposition - pharmaceutical composition.

### Prior Art

At present, by a steady increase of nerve system diseases of vascular, traumatic, toxic, infectious, and autoimmune origin, there is no direct reparants specific for nerve tissue. The purpose of this invention was to provide a biologically active substance which has an antihypoxic, tissue-specific protective effect on nerve tissue by extraction of the PPC from nerve tissue of hoofed farm embryos.

The biologically active substance is a PPC with a molecular weight of its constituent protein/polypeptide components ranging from 0.5 to 200 kDa and the minimum content of medium molecular fraction within the range of 20 to 160 kDa not less than 70%, and the pharmcomposition is produced by dilution of the PPC with a pharmaceutically acceptable buffer and addition of detergents, solubilizers, preservatives, and equal nucleotide component concentration presented by a sodium salt of desoxyribonucleic acid with a molecular weight of its constituent polynucleotides ranging from 12 to 1,000 kDa (18-660 base pairs), with the minimum content of medium molecular fraction within the range of 20 to 500 kDa not less than 80%.

The protein/polypeptide complex was obtained by ion-exchange chromatography of supernatant fraction filtrate of centrifugated homogenate of nerve tissue in the presence of buffer, detergents, preservatives, and solubilizers, from hoofed farm embryos of gestation stage from the middle of the first third to the middle of the last third of pregnancy.

The nucleotide fraction of the PPNC pharmcomposition was obtained by alkaline hydrolysis of tissue homogenate centrifuge effluent remaining after production of the protein/polypeptide fraction with further purification and precipitation of the target fraction with ethanol. The resulting fractions are associated in equal concentration ratios, making the PPNC, adding a pharmaceutically acceptable buffer, detergents, solubilizers, and preservatives.

A number of biologically active substances and preparations are known of peptide and nucleotide nature, made from of raw materials of animal origin, having regenerative and protective activity and used for treatment of nervous system diseases:
A method is known for producing a medication to be used against diseases accompanied with the central nervous system dysfunction [1]. With this method, peptides and proteins with the final concentration of up to 12 mg/ml and with the undetermined spread of molecular weights can be produced from animals' embryo brains using a long-lasting (up to 30 days) extraction followed by enzymatic proteolysis, sediments formed being removed at all stages of precipitation. In this method, proteins and polypeptides produced have not been characterized using the standard physicochemical protein analysis (mass, optical density, phoresis, etc.), whereas the long-lasting extraction and enzymatic proteolysis allow anticipating the low numbers of the molecular weights of the proteins produced and a rather high percentage of admixtures, such as lipoproteins and peptidoglycans, which steadily results in increasing the immunizing power of the medication;

The method is known for treating central nervous diseases [2]. With this method, the extract of proteins and nucleic acids are produced from human embryo brain tissues under standard methods, followed by their sterilization and lyophilic drying, using the lyophilizate mixed with the donated embryo brain. In this method, proteins and polypeptides produced have not been characterized using the standard physicochemical protein analysis (mass, optical density, phoresis, etc.), whereas the initial lyophilic tissue drying changes the quaternary and the tertiary structure of protein/polypeptide components, which steadily results in decreasing the biologic activity and the pharmacological effect specificity of the extract produced, as well as hampers its standartization;
The method is known for manufacturing biologically active preparations from embryo tissues [3]. With this method, a mixture can be produced that does not contain proteins, from the low-molecular thermostable substances of indeterminate composition with the molecular weight of below 10 kDa, containing peptides as 1-3 mg/ml and having the amount of solids as 14-20 mg/ml. The presence of the low-molecular thermo-stable undetermined-composition substances does not allow using this preparation parenterally, whereas the presence of cell wall components and cellular structure membranes determines its high immunizing power;
A group of inventions named *Embryo-Based Medications and Methods for Producing the Same* is known [4]. With this method, the soft tissues of animal embryos mixed with normal saline without specific physical or chemical characteristics are grinded and homogenized. This method does not allow standardizing the mixture obtained, whereas no clear physical and chemical characteristics of the product obtained does not allow introducing this preparation parenterally due to the presence of cell wall and cell structure membrane components, which defines its high immunizing power;
A method is known for producing biologically active preparations based on embryo tissues [5], this method provides for the homogenate hydrolysis at the temperature of +4°-+45° C within 2-42 days, followed by further heating at +60°-+120°C, separating denatured substances, and isolating the thermo-stable undetermined-composition target product with the average molecular weight below 10 kDa from the hydrolyzate.

With this method, the end product contains modified, denatured products of protein and polypeptide hydrolysis with modified quaternary and tertiary structure, and many active molecules decay products, which defines its low biological activity.

A method is known for producing a biologically active preparation from bovine embryo spleen and a pharmaceutical composition based on this preparation, having immunomodulating, antihypoxic, and reparative activity [6]; this method allows for obtaining a mixture of low-molecular peptides, amino acids, nucleic acids derivatives, and other components weighing 5-50 kDa from bovine embryo spleen by 0.9% sodium chloride extraction of homogenate with further sequential ultrafiltration. In this method, resulting proteins, polypeptides and low-molecular nucleic acids are not characterized by standard physical and chemical methods (absorbance, phoresis, etc.); the primary normal saline extraction does not allows for obtaining a wide range of active compounds and a high admixture content, represented by lipoproteids and peptidoglycanes, inevitably results in reduction of biological activity and specificity of the pharmacological effect of the produced preparation, increase of it immunological potency, and hampers its standartization.

A method is known for producing a medication from animal embryo brain tissue and a pharmaceutical composition based on this medication [7]. This method allows for producing a mixture of peptides, amino acids, nucleic acid derivatives, and other components from animal embryo brain exposed, upon prethawing, during 24 hours at the temperature of +10° C by two-stage extracting the homogenate with the 9-% potassium sulfate solution, followed by stabilizing with methyl ester of p-hydroxybenzoic acid to the concentration of 0.07 % and then by sequential ultrafiltration aimed at producing the target fraction, the molecular weights of its components ranging from 10 to 100 kDa. The long time span upon tissue thawing prolongs the postmortem proteolysis, which has a negative impact on the qualitative composition of components extracted and steadily results in losing the tissue-specific biological activity, whereas a rather high percentage of impurities, such as tissue breakdown products, increases the immunizing power of the medication and hampers its standartization.

A method is known for producing a preparation [8] that shows a revitalizing activity, in case of brain dysfunction, which allows extracting biologically active polypeptides of alkaline nature from slaughter animals' cerebral cortexes. The preparation is produced by processing the starting material, which is a tissue containing the hemispheric gray matter, with acetone at the temperature ranging from -10° to -4° C and the brain-tissue-to-acetone ratio of 1:5 within 18-30 h, then homogenized. Then the homogenate is extracted with 2-4-% acetic acid solution during 48-72 h at pH=3.5-4.5, over zinc chloride in the concentration of 0.6-1.2 g/l and over the homogenate-to-acetic-acid-solution ratio of 1: (8-4), followed by centrifuging, settling biologically active substances with acetone at the temperature ranging from -3° to -5° C and the supernatant-to-acetone ratio being equal to 1:(7-5); then the sediments obtained being exposed to ion-exchange chromatography using the 'Biocarb' cation exchanger. Resulting from the ion-exchange chromatography, a fraction is obtained that contains alkaline polypeptides having stimulating (or revitalizing, in case of decreased brain activity) action on the cerebral cortex cells. The method described does not allow for extracting acid or neutral polypeptides from cerebral cortex and producing biologically active polypeptides having another action, such as anticonvulsant activity.

A method is known for producing biologically active lipophilic and hydrophilic fractions of pig placenta [9], [10]; the method allows for producing a mixture of peptides, amino acids, oxyacids by chloroform-ethanol and water-ethanol extraction with saline fractions separation, and usage of freon solvents to produce a lipophilic fraction. This method does not allow for producing nerve tissue-specific proteins and polypeptides, making a systemic effect, and the presence of growth factors without differentiation factors and signaling tissue molecules dramatically increases the risk of neoplastic processes, especially in absence of polynucleotides making a pronounced immunomodulating effect. The resulting fractions have no clear physical and chemical characteristics, and the chloroform-ethanol and water-ethanol extraction method itself provides for production of only low-molecular polypeptides and proteins with modified quarternary and tertiary structure, which inevitably leads to reduction of its specific biological activity.

The closest to the subject group of inventions, from the point of view of the results achieved and some biological activity effects, is the known method for production of a biologically active complex that provides neuroregeneration and neuroprotection [11]. This method allows for obtaining a mixture of proteins and polypeptides from farm animals' embryo brain taken at a certain gestation period (from the beginning of the middle third to the middle of the last third of pregnancy) by the method of anion-exchange chromatography using for application to the equilibrated column a preliminarily filtered tissue homogenate supernatant with a buffer having pH 7.2 - 8.4; the target fractions are produced by separation of proteins and polypeptides associated with the carrier using 0.05M TRIS Glycine buffer solution as a mobile phase, containing 0.1 mM EDTA and 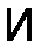 1M NaCl, increasing the concentration by stepwise gradient with a 5% increment. The above complex then undergoes ultrafiltration on a 5 kDa membrane and is desalinated. The target protein/polypeptide fraction characteristics are tested by UV spectrometry, gel-chromatography, denaturing polyacrylamide gel electrophoresis, and amino acid analysis methods. The above biologically active complex has an ultraviolet spectrum absorption peak at wavelength of 280 + 5 nm and a constituent protein weights ranging from 5 to 110 kDa.

Usage of 1M NaCl within the mobile phase at the stage of anion-exchange chromatography for producing this complex generates an excessive ionic strength of the mobile phase equal to 1 mole per litre, and inclusion of EDTA and TRIS into the composition increases it still more. In this connection, the obtained target fraction contains a great amount of acidic highly charged proteins and polypeptides, having in brain tissues cells mainly membrane localization, performing mainly structural functions and only to a small extent regulatory functions, not having tissue-specific nor even expressed biological activity. Further gradient increase of salt concentration with 5% increment still increases the presence of the said proteins, increasing the mixture's biological inactivity, by this reducing its activity and increasing its allergenic potency.

The following pharmaceuticals are also included in the state of the art:
An immune response modifier is known named Derinat, produced by ZAO Pharmaceutical Company "Technomedservis" (Russia) [12], making an impact on cell-mediated 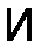 antibody-mediated (humoral) immunity, stimulating reparative processes and hematopoiesis (normalizing the count of granulocytes, lymphocyte, thrombocytes), making an anti-inflammatory and antineoplastic effect, normalizing tissue condition in cases of dystrophic changes of vascular origin, making a slight anticoagulative effect.

A hemorrhagic/ischemic stroke medication Cerebrolysin manufactured by Ebewe (Austria) is known [13], which is the product of intact pig brain processing.

A medication for diseases accompanied with central nervous system dysfunctions, Cerebrocurin manufactured by OOO NIR (Ukraine) is known [14], which is the product of animal embryos' brain processing.

A preparation of polypeptide nature, Cortexin manufactured by OOO Gerofarm (Russia), produced by extracting from bovine and pig brain is known [15], which has nootropic, cerebroprotective, anticonvulsant and antioxidant actions.

A preparation increasing the brain tissue resistance against intoxication, hypoxia, hypoglycemia, mechanical injuries, Cerebrolysat manufactured by Microgen NPO FGUP (Immunopreparat) (Russia), is known [16], which has a nootropic action and represents a bovine brain hydrolyzate.

Derinat polynucleotide composition in the form of sodium salt of desoxyribonucleic acid (Sodium deoxyribonucleate) designates the product's main systemic effect as an immunomodulator impacting cell-mediated 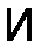 antibody-mediated immunity. The preparation stimulates reparative processes and hematopoiesis (normalizing the count of granulocytes, lymphocyte, thrombocytes), making an anti-inflammatory and antineoplastic effect, normalizing tissue condition in cases of dystrophic changes of vascular origin, making a slight anticoagulative effect. In cases of leukopaenia resulting from polychemotherapy or radiological therapy, leukopoiesis stimulation is observed even after a single injection. Usage of the preparation within the first 24 hours upon radiation exposure speeds up the start and the rate of stem cells, myeloid, lymphoid, and thrombocytic hematopoietic lineages repair. The preparation activates antiviral, antimycotic and antimicrobial immunity, increases phagocyte activity to Chlamydiaceae, Staphylococcus aureus, Escherichia coli, Helicobacter pylori. Derinat significantly reduces cell sensitivity to the damaging effect of cytotoxics and radiotherapy, which shows itself in reduction of cardio- and myelotoxicity in oncology patients and the therapeutic effect stability in retreatment. The preparation makes a non-specific reparative and regenerative effect. The disadvantage of the known product is its low specificity accounted for by the low neuroprotective and regenerative effect, including the reparative potential for nerve tissue.

Cerebrolysin contains amino acids (∼85%), low-molecular peptides (∼15%), trace substances, all obtained from pig brain. Neuroprotective and trophic actions of the preparation are determined by specific peptides and amino acids with molecular weights that do not exceed 10,000 Daltons, from which alanine, leucine and lysine are dominant and determining the preparation features. The preparation is intended for intramuscular and intravenous introduction and has a low concentration of biologically active components, this is why it can be introduced in patients in significant doses and within a long time period. A disadvantage of the known preparation is a significant duration of treatment, low activity and specificity of the preparation due to its low neuroprotective action, because it has an extremely low regenerative, including reparative, potential for nervous tissues.

Cerebrocurin contains water-soluble prenatal neuropeptides and the cleavage products of inactive high-molecular precursors from the animal embryos' brain homogenizate, which, upon dilution with normal saline, is exposed to the interaction with the immobilized proteolytic enzyme, the interaction mode being set on the basis of the check sample of the medication being obtained, and the solution obtained being exposed at a temperature not exceeding +10° C within 30 days. This technology allows for reaching a higher, as compared to Cerebrolysin, activity of the medication produced and its spectrum of action is significantly expanded due to the higher mass of post-proteolysis peptides and low-molecular proteins contained in it. However, regardless of a long period of forming the solution till the end of aggregation and proteolysis processes, this technology does not allow for obtaining a fraction of the cleanness grade necessary for administering the preparation intravenously, which significantly hampers its bioavailability. Water extraction without buffering the solution, without detergents, proteolysis inhibitors or solubilizers at the initial stages of producing Cerebrocurin, as well as the indefinite periods of gestation of the animal embryos' brain, do not allow for providing the necessary standartization degree, which allows for significant dispersion in the composition of evolutionarily fixed protein-peptide concentration ratios obtained, decreasing its tissue-specific neuroregenerative activity and the stability of pharmacology action, limiting by the feed-back biological action due to the signal function of the tissue proteolysis products.

Cortexin contains a complex of water-soluble biologically active alkaline, acid and neutral polypeptides having molecular weights ranging from 0.5 to 15 kDa and the isoelectric point of 3.5-9.5, obtained from farm animals' brain tissues grinded and chilled, by extracting with the acetic acid solution containing zinc chloride, detaching sediments, processing supernatant fluid with acetone, washing the sediments with acetone, drying followed by purification, sterilizing, and freeze-drying of the target product. The preparation is intended for intramuscular introduction and has a low concentration of biologically active low-molecular components, which allows introducing it into patient in significant doses during long periods of time. Although the preparation is involved into regulating the inhibitory-amino-acid-to-excitatory-amino-acid ratios, the concentrations of serotonin and dopamine, having GABA-positive effect, reduces toxic effects of neurotropic agents, improves learning and remembering processes, accelerates the brain function restoration after stresses, Cortexin's activity and specificity are not high due to its weak neuroprotective action, it also has a low regenerative, including reparative, potential for nervous tissues, which makes treatment with this preparation longer.

Cerebrolysat also contains water-soluble polypeptides resulting from the hydrolysis of bovine brain tissues, having low enzymatic activity, with molecular weight non-exceeding 15 kDa. The preparation amino-acid composition variation given in the patent allows a significant dispersion in the composition of the peptide concentration ratios obtained, which defines the low tissue-specific activity and the stability of pharmacological action with low neurometabolic and unexpressed nootropic effects.

### Disclosure of Invention

The objective of this invention is production of a biologically active substance and a pharmcomposition based on it, with enhanced properties and a high biological activity, making skin tissue-specific reparative and regenerative effect on nerve tissue, and development of a method for producing the same by extraction of a biologically active protein-polypeptide complex and polynucleotides for producing a pharmcomposition from nerve tissue of hoofed farm embryos, which objective is solved through a selected set of procedures and modes. Particularly important was to determine the optimal gestation timing of fetal nervous tissue, as well as selection of reagents, their dosage, extraction and buffering regimes, and regulation of proteolytic activity, to maintain effective concentration ratios of proteins and polypeptides fixed evolutionarily and defining the biological activity of the complex, providing reparative and regenerative tissue-specific effect, and polynucleotides for production of the pharmcomposition. The claimed group of inventions includes the protein/polypeptide complex, a method for producing the same from nerve tissue of hoofed livestock embryos at a certain gestation stage, and a pharmcomposition based on this protein/polypeptide complex.

Now therefore, the goal set is achieved by the new protein/polypeptide complex (PPC), possessing antihypoxic, tissue-specific reparative action on the central and peripheral nervous system, derived from the embryonic nervous tissue of hoofed livestock within the gestation period from the middle of the first third till the middle of the last third of pregnancy according to the method according to the invention, including tissue-specific negatively charged slightly acidic, neutral proteins and peptides relative to the growth factors, signaling molecules, determining its biological and pharmaceutical activity, with molar weights from 0.5 to 200 kDa, while at least 70% of the total protein weight has molar weights within the range of 20 to 160 kDa, possessing specific set of stripes at the SDS-polyacrylamide gel electrophoresis («SDS-Page») in 5% polyacrylamide gel in contrast to the standard set of marking proteins with the molar weight range from 1 kDa to 250 kDa and absorption peak at the wavelength 215±5 nm upon removal of UV-spectrum in the wave length range from 200 to 500 nm.

The goal set is also achieved with a new derivation method of protein/polypeptide complex possessing antihypoxic, tissue-specific reparative action on central and peripheral nervous system, in which nerve tissue of hoofed livestock embryos within the gestation stage from the middle of the first third till the middle of the last third of pregnancy are stepwise:
- homogenization in a buffer with a simultaneous extraction in presence of reversible proteolysis inhibitors and nonionic detergents at pH of at least 5.2 and not higher than 8.5, with further centrifugation with a g-value ranging within the rangeof 10.000 - 28,000 during 90 - 30 min, respectively;
- the obtained supernatant is filtered;
- the filtrate is subjected to anion-exchange chromatography on the column with anion-exchange carrier proteins and peptides connected with the carrier are separated, using a buffer solution as a mobile pahse initially having ionic strength (I) not higher than 0.1 mmol/L, increasing it gradually or stepwise by step 0.025 mmol/L and starting collection of target fractions with the eluent ionic strength from 0.125 to 0.15 mmol/L with pH from 5.2 to 8.5;
obtained fractions are combined and subject to sterilization filtration.

Another invention of the claimed group is a pharmaceutical formulation possessing antihypoxic, immune modulating, tissue-specific reparative action on the nervous tissue, prepared in the form of a solution containing a biologically active protein/peptide complex according to claim 2 in concentration of 0.05 - 2.0 mg/ml, obtained using the method according to claim 1, and polynucleotide fraction, derived by alkaline hydrolysis of embryonic tissue homogenate centrifugate, left after derivation (separation) of protein-peptide fraction in the form of protein-peptide complex with further purification and precipitation of the target fraction with ethanol, derived by the method according to claim 1, in the form of DNA sodium salt, demonstrating specific immune adjusting activity combined with the protein-peptide fraction, in at least equal content with the protein-peptide complex, and a pharmaceutically acceptable diluent.

As a pharmaceutically acceptable diluent the pharmaceutical composition may contain a pharmacopoeial buffer and, possibly, excipients including high-molecular compounds, stabilizers, preservatives, and solubilizers.

The pharmaceutical formulation contains polynucleotide cut in the form of medium and low molecular cut of DNA sodium salt - from 12 to 660 kDa (18 - 1000 base pairs) having maximum absorption UV-spectrum at the wave length of 260±2 nm and the minimum one - at the length of 230±2 nm in the zone of wave lengths from 190 to 325 nm with the ratio D260/280 from 1.6 to 2.0, presence of characteristic specific stripes upon electrophoresis in 1.8% agarose gel upon coloring with ethydium bromide, in contrast to the standard set of markers with the range of molar weights from 75 to 7000 base pairs and dark blue coloring upon qualitative reaction to ribose, and protein addition, upon Lowry content determination, not higher than 0.18%.The above pharmaceutical composition is adapted for parenteral application -intradermally or subcutaneously, intranasally or contact applicationally to mucous membranes and skin.

Below there is a brief description of the method for producing the complex and the complex itself and the pharmcomposition based on it.

### Preferred Embodiment

Now therefore, the protein/polypeptide complex is produced according to the following procedure (some operations are described with detailing not limiting the method):
- fetal nerve tissue of hoofed farm animals of gestation stage from the middle of the first third to the middle of the last third of pregnancy are defrozen;
- homogenized in a buffer with a simultaneous extraction in presence of reversible proteolysis inhibitors and nonionic detergents at minimum pH=5.2 and maximum pH=8.5, with minimum ratio 1:0.5; - the homogenate is separated from ballast substances by way of filtering through a close fabric with further centrifugation at g ranging from 10,000 - 28,000 during 90 - 30 min; the resulting ballast tissue material after filtering through a close fabric is combined with centrifuge effluent in order to produce the nucleotide fraction of the pharmcomposition.

### Production of a protein/polypeptide complex:

- the supernatant is filtered through a membrane filter with 0.45 µm pore diameter;
- the filtrate is applied to an equilibrated chromatographic column with an anion-exchange carrier, e.g. Toyopearl DEAE-650M;
- the proteins associated with the carrier are separated by a smooth or stepwise gradient of the solution ionic strength, using as a mobile phase a buffer solution with maximum ionic strength of 0.1 mmol/l increasing it with a 0.025M increment
- the filtrate is applied to an equilibrated chromatographic column with an anion-exchange carrier, e.g. Toyopearl DEAE-650M;
- the proteins associated with the carrier are separated by a smooth or stepwise gradient of the solution ionic strength, using as a mobile phase a buffer solution with maximum ionic strength of 0.1 mmol/l increasing it with a 0.025M increment and starting to collect target fractions using a buffer with ionic strength ranging from 0.125 to 0.15 mmol/l with pH ranging from 5.2 to 8.5;
- the target fractions are associated and undergo ultrafiltration in a plant at backpressure not exceeding 8.0 kgf/cm² through materials with retention potential exceeding 200 kDa; diluted with, for example, 0.05M glycine-NaOH at pH=7.4 to the total protein concentration equal to 1.0 - 2.0 mg/ml;
- the resulting solution undergoes sterilization filtration through a membrane filter with maximum pore diameter 0.22 µm, and the total protein concentration is defined.

All preparatory processes and operations of obtaining the protein/polypeptide fraction are preferably performed at a temperature ranging from +2° to +8°C.

In order to identify the produced protein/polypeptide complex, UV spectrometry, gel-chromatography, and polyacrylamide gel electrophoresis methods are used. The ultraviolet spectrum is recorded at wavelengths ranging from 200 to 500 nm: the absorption peak appears at wavelength of 215 ± 5 nm (Picture 1.). The constituent protein molecular weight is defined by denaturing gel electrophoresis («SDS-Page») in 5% polyacrylamide gel compared to the standard set of marker proteins having molecular weights ranging from 1 kDa to 250 kDa (Picture 2.). The complex has the isoelectric point value ranging from 4.2 to 8.4. The preparation includes proteins with molecular weights ranging from 0.5 to 200 kDa, among which at least 70% have molecular weights ranging from 20 to 180 kDa.

### Production of a polynucleotide fraction for the pharmcomposition and of the pharmcomposition itself:

- associated ballast tissue material and centrifuge effluent of filtered homogenate, are resuspended with a mixer in a solution containing 2.5 mM EDTA and 25 mM sodium citrate with pH 7.0 - 8.0, within the same temperature range in minimum ratio 1:1, incubating the resulting mass for 30 min;
- at the hydrolysis stage, 10M NaOH is added to the resulting mass based on the maximum final alkali molar percentage 2 and, upon stirring, the mixture is incubated for 16 hours at +48°C;
- the mixture is cooled down to +2°C and centrifugated at ∼32,000 g and +4°C for 40 min;
- before supernatant transfer, a thin yellow oil/fatty film is removed from the supernatant surface in centrifugal bottles using filter paper straps;
- 1/10 of 10M NaOH volume is added to purified associated supernatant for second hydrolysis and incubated at +48°C for 5 hours;
- the resulting dihydrolyzate is cooled on ice bath (moist ice, 0°C), then, at continuous agitation and under pH meter control, glacial acetic acid is added to the mixture pH not exceeding 5.4, observing setting of white voluminous sediment of denatured proteins;
- the mixture is centrifugated at ∼32,000 g and +4°C for one hour;
- 0.6 of isopropanol volume is added to the resulting supernatant;
- the resulting mixture, upon incubation at room temperature for 20 min, is centrifugated at ∼32,000 g and +20°C for 1 hour, forming DNA sediment;
- the sediment is diluted in 100 ml H₂O, and 1/10 of 3M sodium acetate volume and 3 ethanol volumes cooled to -20°C are added and agitated;
- the resulting suspension is centrifugated at ∼32,000 g and +4°C for one hour \;
- the resulting sediment is diluted in 50 ml H₂O on magnetic stirrer;
- in the final solution upon sterilization filtration through a membrane filter with maximum pore diameter 0.22 µm, and the polynucleotide fraction concentration is defined.

In order to identify the produced pharmcomposition polynucleotide fraction, UV spectrometry and gel electrophoresis in 1.8% agarous gel, as well as qualitative desoxyribose and sodium tests are used. The ultraviolet spectrum is recorded at wavelengths ranging from 190 to 325 nm: the absorption peak appears at wavelength of 260 ± 2 nm (Picture 3). D_{260/280} ratio ranges from 1.6 to 2.0. The constituent nucleotides molecular weight is defined by gel electrophoresis in 1.8% agarous gel at ethydium bromide staining, compared to the standard set of marker proteins having molecular weights ranging from 75 to 7,000 base pairs: the presence of indicative specific bands ranging from 12 to 660 kDa is defined (Picture 4). The qualitative ribose test results in blue staining and an admixture of protein, defined according to Lowry method, not exceeding 18%.

The produced protein/polypeptide complex and nucleotide fraction identification is performed, then, in order to produce the pharmcomposition, they are associated at least in equal parts by concentration with calculation of final total protein and polypeptide concentration in the obtained solution within the range of 0.05 - 2.0 mg/ml at dilution with a pharmaceutically acceptable diluent, e.g. 50mM glycine-phosphate, containing, if necessary, excipients, e.g., with concentrations of 0.5% mannitol, 0.0005% Polysorbate-80 and disodium hydrogen phosphate to pH not below 5.2 and not exceeding 8.5. Repeated identification and filtration sterilization are performed.

The pharmcomposition (PPNC) identifying characteristics are defined by UV spectrometry and gel electrophoresis in 1.8% agarous gel. The ultraviolet spectrum is recorded at wavelengths ranging from 200 to 500 nm: the absorption peak appears at wavelength of 260 ± 2 nm, minimum absorption - at wavelength of 230 ± 2 nm (Picture 5). At electrophoresis in 5% polyacrylamide gel at argentic nitrate staining, the presence of indicative specific protein and nucleotide bands is defined (Picture 6), and at Coomassie brilliant blue staining, only protein fraction characteristic bands are present (Picture 7).

Therefore, for the PPC extraction it is essential:
- to use as the raw material nerve tissue of hoofed farm embryos of gestation stage from the end of the first third to the middle of the last third of pregnancy;
- that detergents and solubilizers were present in the buffer in sufficient concentrations;
- that buffer media pH was falling within the range of 5.2-8.5 at homogenization and anion-exchange chromatography stages;
- that during anion-exchange chromatography the ionic strength (I) of the eluent at the moment of separation of proteins and polypeptides associated with the carrier was not below 0.1 mmol/L, and the target fractions were collected with an eluent with ionic strength not exceeding 0.15 mmol/L;
- that in the described PPC the content of proteins with 20 kDa to 160 kDa molecular weights was at least 70% of the total protein content;
- that while indentifying the claimed PPC and performing denaturing gel electrophoresis («SDS-Page») in 5% polyacrylamide gel compared to the standard set of marker proteins, characteristic bands were present confirming and characterizing the complex protein/polypeptide constituents spectrum. In case of other production parameters, the protein/polypeptide component composition and its constituents ratios will differ.

The claimed biologically active protein/polypeptide complex is fundamentally different from any known biologically active substances produced from animal material by:
- raw material - nerve tissue of farm embryos are used, of a certain gestation stage from the middle of the first third to the middle of the last third of pregnancy;
- methods for production -presence in the extracting (?) buffer detergents and solubilizers and absence of denaturing and degradable compounds - spirits, alkalies, acids, and ferments, allow for obtaining membrane, intercellular, cytoplasmic, nuclear proteins and polypeptides with preserved tertiary structure;
- usage within the mobile phase of millimolar cation concentrations in order to create a moderate ionic strength, by this allowing for obtaining a large amount of nerve tissue specific low charged proteins and polypeptides performing regulatory, signaling, and enzymatic functions, defining a pronounced regenerative and reparative biological tissue-specific activity;
- qualitative composition as a certain fraction of tissue (membranes, cellular, cytoplasmic, nuclear) hydrophilic, low charged proteins and polypeptides, with a concentration ratio of growth and differentiation factors and signaling molecules with molecular weights ranging from 0.5 kDa to 200 kDa, evolutionarily fixed in the ontogenesis;
- identification and standardization of the target fraction obtained - presence of specific bands, characteristic of this complex, in course of electrophoresis in 5% polyacrylamide gel (Picture 6), and removal of molecules with molecular weights exceeding 200 kDa using membrane filtering;
- purity - the resulting protein/polypeptide complex does not contain admixtures such as lipids, peptidoglycanes, lipopolyproteins, carbohydrates, polysaccharides and other compounds;
- administration - parenteral (intradermal, subcutaneous), to mucous membranes (intranasal, subconjunctival) and skin;
- administration safety (absence of toxic, mutagenic, cancerogenic and allergenic effects);
- biological and pharmacological activity (reparative and regenerative) and its specificity (tissue- and organo-specificity).

Hydrolysis parameters change in production of polynucleotide fraction for PPNC pharmcomposition will inevitably result in the pharmcomposition parameters due to polynucleotide components, however, if the recommended concentrations are used, these changes will only insignificantly impact the entire pharmcomposition biological activity. In case of a different raw component for production of the polynucleotide fraction (animal tissues, commercial fish milts, etc.), with production techniques, claimed concentration ratios with the protein/polypeptide complex preserved, and compliance of the resulting fraction with claimed criteria, the biologic activity of the PPNC, as well as that of the pharmcomposition, do not change significantly.

The claimed pharmcomposition in form of PPNC with a pharmaceutically acceptable diluent helps increase the PPC's reparative and regenerative capacity through the specific effect of medium- and low-molecular polynucleotides on antibody-mediated and cell-mediated immunity activation in maximum dosage of 0.1 - 10 mg, and increase the entire composition solution stability and its effect and shelf life.

The invention is illustrated with the following examples without limitation to its scope.

### Example 1. Method for producing the PPC.

### Production of a protein/polypeptide complex from nerve tissue of sheep embryos

440 g of nerve tissue of sheep embryos taken at gestation stage of 16 - 18 weeks is defrozen and homogenated in 1,200 ml of 0.05M TRIS glycine-phosphate buffer solution with pH 8.0, containing 1 mM of EDTA and 0.1% of mannitol, for 5 minutes at +4°C, the homogenate is separated from ballast substances by filtering through a close fabric with further centrifugation at g=10,000 for 90 min, separating the centrifuge effluent. The supernatant is filtered through a membrane filter with 0.45 µm pore diameter, the filtrate is applied to a 200 ml chromatographic column equilibrated with 4 volumes of 0.05 M glycine-phosphate buffer with pH=8.5, with an anion-exchange carrier Toyopearl DEAE-650M; the proteins associated with the carrier are separated by stepwise gradient, using the same buffer solution containing 0.05 M KCI (I = 0.1 mmol/l), increasing salt concentration with 0.025M (I = 0.025 mmol/l) increment and starting to collect target fractions with salt concentrations ranging from 0.075 M (I = 0.125 mmol/l) to 0.1 M (I = 0.15 mmol/l); the target fractions are associated and undergo ultrafiltration in a plant at backpressure not exceeding 8.0 kgf/cm² through materials with retention potential exceeding 200 kDa and diluted with 0.05M glycine-NaOH at pH=8.5; the resulting solution undergoes sterilization filtration through a membrane filter with maximum pore diameter 0.22 µm, and the total protein concentration is defined.

All preparatory processes and operations of obtaining the protein/peptide fraction are performed at a temperature ranging from +2° to +8°C.

The resulting protein/polypeptide complex characteristics:
- the preparation solution ultraviolet spectrum is recorded at wavelengths ranging from 200 to 500 nm: the absorption peak appears at wavelength of 215 ± 5 nm (Picture 1.);
- the constituent protein molecular weight is defined by denaturing gel electrophoresis («SDS-Page») in 5% polyacrylamide gel compared to the standard set of marker proteins having molecular weights ranging from 1 kDa to 250 kDa, the fraction includes proteins with molecular weights ranging from 0.5 to 200 kDa, among which 76% have molecular weights ranging from 20 to 160 kDa (Picture 2);
- the amount of proteins is defined according to Lowry method (without presedimentation) - 2.4 mg/ml;
- amino acid analysis (%): Asp - 10,82 ± 2,8; Thr - 5,4 ± 1,6; Ser - 5,2 ± 1,8; Glu -16,2 ± 3,4; Pro - 7,045 ± 2,5; Gly - 5,2 ± 2,4; Ala - 5,4 ± 1,8; Val - 7,08 ± 2,9; Met - 2,65 ± 1,4; He - 4,45 ± 1,8; Leu -9,4 ± 2,6; Tyr - 4,02 ± 1,2; Phe - 4,8 ± 1,9; Orn- 0,48 ± 0,2; Lys - 8,48 ± 2,4; His - 2,8 ± 0,9; Arg - 6,52 ± 2,2.

### Example 2. Method for producing the PPC.

### Production of a protein/polypeptide complex from nerve tissue of pig embryos.

300 g of nerve tissue of pig embryos taken at gestation stage of 16 - 18 weeks is defrozen and homogenated in buffer solution with pH=5.2, containing 50 mM of Tris-maleate, 1 mM of (EDTA) and 0.1% of mannitol, for 5 minutes at +2°C. The homogenate is separated from ballast substances by filtering through a close fabric with further centrifugation at g=28,000 for 30 min, respectively, associating the resulting ballast tissue material upon filtering through a close fabric with the centrifuge effluent for production of the nucleotide fraction of the pharmaceutical composition. The supernatant is filtered through a membrane filter with 0.45 µm pore diameter and applied to a 250 ml chromatographic column equilibrated with 4 volumes of maleate buffer with pH=5.2, with an anion-exchange carrier DEAE-Sepharose. The proteins associated with the carrier are separated by smoothly increasing the gradient of the ionic strength of the maleate buffer solution with pH=5.2 containing 0.05M NaCl (I = 0.1 mmol/l), starting to collect target fraction with 0.075 M (I = 0.175 mmol/l) and finishing at 0.1M (I = 0.15 mmol/l) salt concentrations; the target fraction undergoes ultrafiltration in a plant at backpressure not exceeding 8.0 kgf/cm² through materials with retention potential exceeding 200 kDa, is desalinated by dialysis against 50 mM of aspartate-NaOH solution, pH 8.5. The solution undergoes sterilization filtration through a membrane filter with maximum pore diameter 0.22 µm.

In order to characterize the produced PPC, UV spectrometry, gel-chromatography, polyacrylamide gel electrophoresis, and amino acid analysis methods are used. The ultraviolet spectrum is recorded at wavelengths ranging from 200 to 500 nm: the absorption peak appears at wavelength of 215 ± 5 nm (Picture 8.). The constituent protein and polypeptide molecular weights are defined by denaturing gel electrophoresis («SDS-Page») in 5% polyacrylamide gel compared to the standard set of marker proteins having molecular weights ranging from 1 kDa to 250 kDa (Picture 9). The preparation includes proteins with molecular weights ranging from 0.5 to 200 kDa, among which 72% have molecular weights ranging from 20 to 160 kDa. The amount of proteins in the resulting PPC is defined according to Lowry method (without presedimentation) - 1.85 mg/ml.

Amino acid analysis of the resulting complex (%): Asp - 8,3 ± 2,7; Thr - 6,9 ± 2,1; Ser - 6,2 ± 1,5; Glu -12,9 ± 4,7; Pro - 7,05± 2,4; Gly - 8,2 ± 2,1; Ala - 6,1 ± 1,4; Val - 7,5 ± 2,6; Met - 1,4 ± 0,7; He - 4,9 ± 1,2; Leu - 8,7 ± 2,3; Tyr - 5,4 ± 1,8; Phe - 5,5 ± 2,1; Orn- 0,6 ± 0,1; Lys - 8,6 ± 2,8; His - 1,7 ± 0,9; Arg - 7,1 ± 2,8.

### Example 3. Method for producing the pharmcomposition

*a) production of the polynucleotide fraction:*
   - associated ballast tissue material and centrifuge effluent of filtered homogenate weighing 280 g, are resuspended with a mixer in a solution containing 2.5 mM EDTA and 25 mM sodium citrate with pH 7.8, within the same temperature range in volume ratio 1:1.5 (650 ml), incubating the resulting mass for 30 min; 10M NaOH is added to the resulting mass based on the maximum final alkali molar percentage 2 (158 ml) and, upon stirring, the mixture is incubated for 16 hours at +48°C.
   - the mixture is cooled down to +2°C and centrifugated at ∼32,000 g and +4°C for 40 min, removing, before supernatant transfer, a thin yellow oil/fatty film from the supernatant surface in centrifugal bottles using filter paper straps.
   - 1/10 of 10M NaOH (58 ml) volume was added to purified associated supernatant for second hydrolysis and incubated at +48°C for 5 hours.
   - the resulting dihydrolyzate was cooled on ice bath (moist ice, 0°C), then, at continuous agitation and under pH meter control, glacial acetic acid was added to the mixture pH not exceeding 5.4 (63 ml), observing setting of white voluminous sediment of denatured proteins.
   - the mixture was centrifugated at ∼32,000 g and +4°C for one hour; 0.6 of isopropanol volume was added to the resulting supernatant; and, upon incubation at room temperature for 20 min, was centrifugated at ∼32,000 g and +20°C for 1 hour, forming DNA sediment.
   - the sediment was diluted in 100 ml H₂O, and 1/10 of 3M sodium acetate volume and 3 ethanol volumes cooled to -20°C were added and agitated.
   - the resulting suspension was centrifugated at ∼32,000 g and +4°C for one hour, diluting the resulting sediment in 50 ml H₂O on magnetic stirrer. In the final solution, upon sterilization filtration through a membrane filter with maximum pore diameter 0.22 µm, the resulting polynucleotide fraction concentration was defined (2.8 mg/ml).

   The resulting nucleotide fraction characteristics:
   - the solution ultraviolet spectrum was recorded at wavelengths ranging from 190 to 325 nm: the absorption peak appeared at wavelength of 260 ± 2 nm, minimum absorption - at wavelength of 230 ± 2 nm (Picture 8) with D_{260/280} ratio from 1.6 to 2.0 (Picture 3);
   - the constituent nucleotide molecular weight was defined by denaturing SDS-Page electrophoresis in 1.8% agarous gel at ethydium bromide staining compared to the standard set of markers having molecular weights ranging from 75 to 7,000 base pairs. The presence of indicative specific bands within the range of 12 to 660 kDa is noted (Picture 4). At qualitative ribose test, blue staining appears. Protein concentration in the extracted nucleotide fraction, defined according to Lowry method, was 0.16 mg/ml (i.e. ∼0.15% of DNA).
b) The PPC solution produced according to Example 1 is associated with polynucleotide fraction solution presented by a sodium salt of desoxyribonucleic acid produced by a known method described, for instance, in Example 3, under the condition that the final total protein concentration (defined according to Lowry method) and polynucleotide concentration (defined by spectrophotometry), upon dilution with 50 mM of glycine-phosphate buffer with possible addition of excipients, is 2.0 mg/ml. Excipients may be added to the produced PPNC: detergents, solubilizers, preservatives and high-molecular compounds (taking into account the PPNC final concentration), in particular, up to 0.5% mannitol, 0.0005% Polysorbate-80, 0.03% Nipagin and disodium hydrogen phosphate with pH up to 8.5. The resulting pharmcomposition undergoes sterilization filtration through a membrane filter with 0.1 µm maximum pore diameter, is bottled to aseptic glass bottles and sealed.

The resulting PPNC pharmcomposition characteristics:
- the protein/polypeptide/nucleotide complex identifying characteristics, upon association of produced protein and nucleotide fractions in equal parts, are defined by UV spectrometry and gel electrophoresis in 1.8% agarous gel, the ultraviolet spectrum is recorded at wavelengths ranging from 200 to 500 nm: the absorption peak appears at wavelength of 260 ± 2 nm, minimum absorption - at wavelength of 230 ± 2 nm (Picture 5). At electrophoresis in 5% polyacrylamide gel at argentic nitrate staining, the presence of indicative specific protein and nucleotide bands is defined (Picture 6), and at Coomassie brilliant blue staining, only protein fraction characteristic bands are present (Picture 7).

### Example 4. Method for producing the pharmcomposition

*a) Production of the polynucleotide fraction*
   - ballast tissue material and centrifuge effluent of filtered homogenate weighing 175 g, are resuspended with a mixer in a solution containing 2.5 mM EDTA and 25 mM sodium citrate with pH 7.8, within the same temperature range in volume ratio 1:3 (425 ml), incubating the resulting mass for 30 min; 10M NaOH is added to the resulting mass based on the maximum final alkali molar percentage 2 (112 ml) and, upon stirring, the mixture is incubated for 16 hours at +48°C;
   - the mixture is cooled down to +2°C and centrifugated at ∼32,000 g and +4°C for 40 min, removing, before supernatant transfer, a thin yellow oil/fatty film from the supernatant surface in centrifugal bottles using filter paper straps;
   - 1/10 of 10M NaOH volume (42 ml) was added to purified associated supernatant for second hydrolysis and incubated at +48°C for 5 hours;
   - the resulting dihydrolyzate was cooled on ice bath (moist ice, 0°C), then, at continuous agitation and under pH meter control, glacial acetic acid was added to the mixture pH not exceeding 5.4 (48 ml), observing setting of white voluminous sediment of denatured proteins;
   - The mixture was centrifugated at ∼32,000 g and +4°C for one hour; 0.6 of isopropanol volume was added to the resulting supernatant; and, upon incubation at room temperature for 20 min, was centrifugated at ∼32,000 g and +20°C for 1 hour, forming DNA sediment.
   - the sediment was diluted in 100 ml H₂O, and 1/10 of 3M sodium acetate volume and 3 ethanol volumes cooled to -20°C were added and agitated;
   - The resulting suspension was centrifugated at ∼32,000 g and +4°C for one hour, diluting the resulting sediment in 50 ml H₂O on magnetic stirrer. In the final solution, upon sterilization filtration through a membrane filter with maximum pore diameter 0.22 µm, the polynucleotide fraction concentration was defined (2.1 mg/ml).

   In order to characterize the produced nucleotide fraction, UV spectrometry and gel electrophoresis in 1.8% agarous gel, as well as qualitative desoxyribose and sodium tests were used. The ultraviolet spectrum is recorded at wavelengths ranging from 190 to 325 nm: the absorption peak appears at wavelength of 260 ± 2 nm, minimum absorption - at wavelength of 230 ± 2 nm (Picture 10). D_{260/280} ratio ranges from 1.6 to 2.0. The constituent nucleotides molecular weight is defined by gel electrophoresis in 1.8% agarous gel at ethydium bromide staining, compared to the standard set of marker having molecular weights ranging from 70 to 7,000 base pairs: the presence of indicative specific bands ranging from 12 to 660 kDa is noted (Picture 11). The qualitative ribose test results in blue staining. Protein concentration in the extracted nucleotide fraction, defined according to Lowry method, was 0.12 mg/ml (i.e. ∼0.14% of DNA).
b) The PPC solution produced according to Example 2 is associated with polynucleotide fraction solution presented by a sodium salt of desoxyribonucleic acid produced by a known method described, for instance, in Example 3, under the condition that the final total protein concentration (defined according to Lowry method) and polynucleotide concentration (defined by spectrophotometry), upon dilution with 50 mM of glycine-phosphate buffer is 0.05 mg/ml. Excipients may be added to the produced PPNC: detergents, solubilizers, preservatives and high-molecular compounds (taking into account the PPC concentration), in particular, up to 0.5% mannitol, 0.0005% Polysorbate-80, 0.005% benzalkonium chloride and sodium dihydrogen phosphate with pH up to 5.2. The resulting pharmcomposition undergoes sterilization filtration through a membrane filter with 0.1 µm maximum pore diameter, is bottled to aseptic glass bottles and sealed.

The resulting pharmcomposition characteristics with reference designation of the PPNC complex produced in it:
- the protein/polypeptide/nucleotide complex identifying characteristics, upon association of produced protein and nucleotide fractions in equal parts, are defined by UV spectrometry and gel electrophoresis in 1.8% agarous gel, the ultraviolet spectrum is recorded at wavelengths ranging from 200 to 500 nm: the absorption peak appears at wavelength of 260 ± 2 nm, minimum absorption - at wavelength of 230 ± 2 nm (Picture 12). At electrophoresis in 5% polyacrylamide gel at argentic nitrate staining, the presence of indicative specific protein and nucleotide bands is defined (Picture 13), and at Coomassie brilliant blue staining, only protein fraction characteristic bands are present (Picture 14).

### Example 5. PPNC effect on brain explants development

The experiments were performed with 52 cortex fragments and 40 cerebrospinal ganglia fragments of 10-12-days chicken embryos. The nutritional medium for explants culture contained 35% of Eagle's solution, 25% of fetal calf serum, 35% of Hanks' solution, 5% of chicken fetal extract; the additives included glucose (0,6%), insulin (0,5 U/ml), penicillin (100 U/ml), glutamine (2 mM). Cortex or cerebrospinal ganglia fragments were placed into this medium and cultured on a collagen-coated surface in Petri dishes in a thermostat at 36.7°C for 2 days. Into the experimental medium, the studied PPNC was added and, as a positive control - Cerebrolysin and Cortexin in the following concentrations: 0.5, 1, 2, 10, 20, 50, 100, 200, 400, 800, 1000 ng/ml. The biological activity criterion was the area index (Al), i.e. the entire explant area including the growth zone to the brain fragment initial area ratio. The significance of difference between the average Al being compared was assesses using a Student's t-test. Al values were expressed in percent; Al reference value was taken as 100%. The reference brain explants growth zozne comprised short neuritis and migrating gliacytes and fibroblast-like cells. In course of study of the preparations' direct effect on the brain fragments, the following series of experiments were conducted. Cerebrolysin and Cortexin in different concentrations were added to the nutritional medium of chicken embryos cortex and cerebrospinal ganglia fragments. On the third day of culturing using Cerebrolysin at 100 ng/ml concentration, a significant increase of cortex explant Al by 24±3.5 % was observed compared to the reference Al values. In other Cerebrolysin concentrations significant cortex explant Al values were not recorded. On the third day of culturing using Cortexin at 50 ng/ml concentration, a significant increase of cortex explant Al by 32±4% was observed compared to the reference Al values. A significant increase of cortex explant Al by 28+3.5% was recorded with Cortexin concentration of 100 ng/ml, by 23+2% with Cortexin concentration of 200 ng/ml. In other Cortexin concentrations significant cortex explant Al values were not recorded. A pronounced stimulation of cortex explant was revealed with the studied PPNC taken in 20 ng/ml concentration, when the experimental explants Al was significantly by 48±4.5% higher than reference explants Al and compared explants with Cortexin and Cerebrolysin effect. When Cerebrolysin was added to cerebrospinal ganglia explants culture medium, no significant explants Al increase was recorded, while adding Cortexin resulted in explants Al increase by 22±3%. When the studied PPNC was added to cerebrospinal ganglia explants culture medium, explants Al increase was observed by 36±3.5%. Studies of cortex and cerebrospinal ganglia explants with longer culture periods - 7 days - showed the same neurite stimulating effects with the same concentrations of the studied complex. Therefore, with regard to brain tissues, effective concentration threshold reduction was recorded for the studied PPNC compared to Cerebrolysin and Cortexin. Thus, cortex explants growth zone increase was observed with Cerebrolysin taken only in 100 ng/ml concentration, Cortexin - only in 50 and 100 ng/ml concentrations, and the studied PPNC - in 20, 10, 50 and 100 ng/ml concentrations respectively. Besides, the PPNC stimulating effect was significantly stronger than that of Cortexin and Cerebrolysin. Cerebrolysin did not make a significant effect on cerebrospinal ganglia explants growth zone increase; Cortexin significantly increased the Al by 24%, while the studied protein/polypeptide complex provided for Al increase by 36%. This reveals a pronounced and specific stimulating effect of the PNC on neurons of different brain divisions.

### Example 6. PPNC pharmcomposition toxicity study [17].

The PPNC general toxical action was studied in acute toxicity regime with a single dosing and subchronic and chronic toxicity with long term complex administration [17]. Acute toxicity was studied in 72 CD-1 male mice weighing 20-22 g. The animals were randomly assigned to 6 equal groups. The complex was introduced intramusculary in a single dose of 5 mg/kg, 10 mg/kg, 100 mg/kg, 200 mg/kg, 500 mg/kg in 0.5 ml of sterile 0.05 M glycine-NaOH solution with pH=7.5. The reference group animals were administered sterile 0.05 M glycine-NaOH solution with pH=7.5 in the same dosage. Subchronic toxicity study was conducted in 95 CD-1 male mice weighing 150-180 g. The experimental animals were subjected to a single subcutaneous PPNC introduction daily for 90 days in the amount of 1 mg/kg, 10 mg/kg, 50 mg/kg, 100 mg/kg in 0.5 ml of sterile 0.05 M glycine-NaOH solution with pH=7.5. The reference group animals were administered sterile 0.05 M glycine-NaOH solution with pH=7.5 in the same dosage. Prior to and on the 30^{th}, 60^{th}, and 90^{th} day upon beginning of introduction, the animals' periferic blood morphological composition and properties analysis was performed. At completion of the experiment, biochemical and coagulologic blood analysis was performed.

Chronic toxicity study was conducted during 6 months in 112 male guinea pigs weighing 250-280 g. The experimental animals were subjected to a single subcutaneous PPNC introduction daily for 6 months in the amount of 1 mg/kg, 10 mg/kg, 50 mg/kg, 100 mg/kg in 0.5 ml of sterile 0.05 M glycine-NaOH solution with pH=7.5. The reference group animals were administered sterile 0.05 M glycine-NaOH solution with pH=7.5 according to the same scheme in the same dosage. The following animals' periferic blood parameters were defined using standard methods: red blood cells, reticulocytes, thrombocytes white blood cells count, Hb level, leucogram, erythrocyte sedimentation rate (ESR), fragility. Apart from that, the blood serum total protein content was defined according to Lowry method, potassium and sodium content was defined by plasma spectrophotometry. Upon completion of the experiment, pathomorphological study of brain and medulla, cerebrospinal ganglia, thyroid, parathyroids, suprarenal glands, testes, hypophysis, heart, lungs, aorta, liver, kidneys, bladder, pancreatic gland, stomach, small intestine, large intestine, thymus, spleen, lymphatic glands, marrow was performed. Acute toxicity study showed that a single dosage of the studied complex, introduced to animals in the dosing exceeding the therapeutic one, recommended for clinical usage, more than thousandfold, does not induce toxic reactions, which is indicative of a high therapeutic index of the said complex.

The complex subchronic and chronic toxicity study revealed absence of side effects in case of its long-term usage in dosage, 600 times exceeding the estimated therapeutic dose. The study of the complex impact on the guinea pigs' periferic blood morphological composition and biochemical parameters in 3 and 6 months after beginning of administration revealed no significant modifications of values.

Assessment of the animals' general medical condition, morphological and biochemical parameters of periferic blood, morphologic state of internal organs, state of cardiovasculare and respiratory systems, liver and kidneys functions showed no abnormal changes in organisms. Therefore, the protein/polypeptide complex produced according to the proposed method, in case of long-term administration to animals, does not have toxic properties preventing it from further usage as a medication for parenteral infusion.

### Example 7. Subchronic toxicity study at intranasal introduction in mature animals

The study was performed in mature female chinchilla rabbits weighing 2,500 g [17]. The animals were taken from a nursery. The rabbits were kept in standard cages 1 animal in each. They were fed with pelletized feed and fresh vegetables. Water and food were freely available to the animals. The vivarium had artificial daylight. The air temperature was +18 - +20° C [18]. There were 3 groups, 5 animals in each. The groups were divided as follows: 1 group (therapeutic dose) - pharmcomposition 0.1 mg/ml 0.2 ml introduced once daily; 2 group - pharmcomposition 0.1 mg/ml 0.2 ml introduced twice daily; 3 group - intact animals. Introduction period was 1 month.

Toxicity was assessed against an observation checklist: by survival rate, general appearance, animals' condition and behavior, body weight variation (the rabbits were weighed at the beginning and at the end of the experiment). In course of the experiment, the local irritant effect was defined by changes of nasal mucosae.

Prior to and at the end of the experiment, experimental animals were subjected to periferic blood morphological composition analysis (red blood cells, white blood cells, thrombocyte counts, Hb level); biochemical values (protein, urea, creatinine, glucose, total cholesterol and triglycerides levels), activity of some plasma enzymes (aspartate- and alanine aminotransferase, alkaline phosphatase, lactate-dehydrogenase). To define the said values, blood was taken from auricular veins in the amount of 1.5-2.0 ml.

Blood corpuscles were counted using an automatic blood analyzer Picoscale (Hungary). Hb level was defined by hemiglobincyanide method. Biochemical value levels were measured with automated system. FP-901, «Labsystems» Finland.

Upon completion of a chronic experiment, the animals were sacrificed using narcosis overdose for pathomorphological study of organs and tissues. The animals were dissected immediately upon their death, according to a full pathoanatomical scheme.

Morphometric organs parameters were estimated using Sartorius scales (Germany) with further calculation of organs weights and their standard deviations.

For pathomorphological studies, fresh tissue samples were gelatinated and cryostated to obtain sections up to 5 µm thick. The tissues then were fixed and stained with hematoxylin-eosin. Microscopical investigation was performed with an Opton microscope (Germany).

The experimental data were compared to the reference data and analyzed using statistic software.

### Examination Results

### 1. Visual observations:

*Survival rate:* all experimental animals survived the pharmcomposition introduction in studied dosing. Deaths were not recorded.

*Behavior*: neither hyper, nor hypoactivity was observed in experimental animals compared to reference groups. Myotonus in experimental and reference animals was not marked with hypererethism. It was noted that the preparation introduction did not induce a sense of discomfort in animals.

*Appearance*: all animals, regardless of the dosing, were medium finish, did not suffer from exhaustion or obesity. Hair was smooth and glossy; hairslip or fragility were not detected. Corneal opacity, tearing or any pathological characteristics were not observed. Pinnae were pink with no crust, not inflamed, jerking was not observed. Teeth were of normal color, breaking was not observed.

*Functions:* experimental animals breathing, like that of reference animals, was quiet, of normal rhythm, unobstructed; salivation with no distress; urinary frequency, amount and color of urina fell within physiological range; excreta did not differ from the reference material in color and consistency.

### 2. Clinical study:

*Body weight dynamics*: positive in all experimental groups; there was no significant difference between experimental and reference intact animals.

*Blood analysis:* in all groups Hb level, red blood cells, white blood cells, thrombocyte counts fell within physiological range.

*Biochemical study*: blood glucose, alanine and asparaginic aminotransferase activity in all groups fell within physiological range. Protein concentration in rabbits receiving the pharmcomposition once and twice daily did not differ statistically.

### 3. Pathomorphological data:

In all tested groups: in internal organs (liver, kidneys, lungs, heart, spleen, stomach, large and small intestine), internal secretion glands (thyroid, thymus, suprarenal capsules, pancreatic gland), reproductive organs (uterus, ovaries), lymph glands, cerebrum, skin, nose and throat mucosae pathomorphological changes were not detected.

As can be seen from the above, the pharmcomposition nasal form subchronic toxicity was studied. The spray subchronic study revealed no significant internal organs changes in experimental animals nor local irritative effect. For subchronic toxicity study, clinical research methods, common hematologic and biochemical blood analysis methods, and histologic examination of internal organs and nasal mucosae were used.

Pharmcomposition subchronic toxicity study at intranasal introduction in immature rabbits.

Study was performed in three weeks old baby chinchilla rabbits weighing 300 to 500 g. The preparation was applied once and twice daily by one spray for two weeks [17]. The animals were taken from a nursery. The rabbits were kept in standard cages 1 animal in each. They were fed with pelletized feed and fresh vegetables. Water and food were freely available to the animals. The vivarium had artificial daylight. The air temperature was +18 - +20° C [18]. There were 3 groups, 5 animals in each. The groups were divided as follows: 1 group (therapeutic dose) - pharmcomposition 0.1 mg/ml 0.2 ml introduced once daily; 2 group - pharmcomposition 0.1 mg/ml 0.2 ml introduced twice daily; 3 group - intact animals. Introduction period was 2 weeks.

Toxicity was assessed against an observation checklist: by survival rate, general appearance, animals' condition and behavior, body weight variation (the rabbits were weighed at the beginning and at the end of the experiment). In course of the experiment, the local irritant effect was defined by changes of nasal mucosae.

Prior to and at the end of the experiment, experimental animals were subjected to periferic blood morphological composition analysis (red blood cells, white blood cells, thrombocyte counts, Hb level); biochemical values (protein, urea, creatinine, glucose, total cholesterol and triglycerides levels), activity of some plasma enzymes (aspartate- and alanine aminotransferase, alkaline phosphatase, lactate-dehydrogenase). To define the said values, blood was taken from auricular veins in the amount of 1.5-2.0 ml. Blood corpuscles were counted using an automatic blood analyzer Picoscale (Hungary). Hb level was defined by hemiglobincyanide method. Biochemical value levels were measured with automated system. FP-901, «Labsystems» Finland.

Upon completion of a chronic experiment, the animals were sacrificed using narcosis overdose for pathomorphological study of organs and tissues. The animals were dissected immediately upon their death, according to a full pathoanatomical scheme.

Morphometric organs parameters were estimated using Sartorius scales (Germany) with further calculation of organs weights and their standard deviations.

For pathomorphological studies, fresh tissue samples were gelatinated and cryostated to obtain sections up to 5 µm thick. The tissues then were fixed and stained with hematoxylin-eosin. Microscopical investigation was performed with an Opton microscope (Germany).

The experimental data were compared to the reference data and analyzed using statistic software.

### Examination Results

*Estimate of irritative effect on nasal mucosa.*

### In-life biomicroscopic study

During the entire observation period the animals preserved normal behavior; meatus discharge and crust formation were not recorded. When drops were used, the rabbits had sneezing reflex which disappeared in 1-2 minutes. In case of instillation of drops, both in the therapeutic group and in the exceeding dosage group, the baby rabbits had mechanical irritation showing as sneezing reflex, nose jerking; the animals rubbed their noses with paws.

*Reference intact group.* The mucosa was rose-pink, without oedemas and discharge. Nasoscope examination revealed no epithelium defects. The nose mucous discharge had a transparent watery look. Reflexes fell within physiological range.

Confocal microscopy: epithelial layer has a densely packed structure of terminally differentiated cells. Cells have gap junctions.

*In the therapeutic dose group:* during the entire observation period, the nose mucosae were clean, rose-pink without discharge, oedemas were not noted. The mucosa vessels were not changed. The epithelium condition was studied on slit illumination. No pathological changes were detected.

*In group x10.* No pathological mucosa changes were detected.

### Clinical and Biochemical studies

Blood analysis: haemoglobin contents, red blood cells, white blood cells, thrombocyte counts in all groups fell within physiological range. Experimental groups data had no significant difference from reference intact groups data and that of the reference substance.

*Biochemical study*: blood glucose, alanine and asparaginic aminotransferase activity in all groups fell within physiological range.

Conclusion. Toxicity study of the original pharmcomposition in subchronic experiment was performed to immature animals.

Intranasal introduction of the preparation both in therapeutic and tenfold dose during two weeks did not result in internal organs changes, nor had a toxic effect on hemic system, nor local irritative effect.

### Example 8. PPC and PPNC specific activity study [17].

Local irritative effect. The studies performed showed that the active PPC substance, when introduced subcutaneously, intravenously, and intranasally, does not make local irritative effect. In case of quick intravenous introduction, hyperthermia effects are possible. In case of intranasal administration to rabbits for one month, the local irritative effect on nasal and throat mucosa was not registered.

### Immunotoxic and allergenic properties study

### Active cutaneous anaphylaxis

The experiment was conducted in 3 guinea pigs groups, 10 animals in each. The animals were sensibilized according to the following scheme: first injection was made subcutaneously, with two intramuscular injections to the hip area to follow with a day's interval. On the 14^{th} day, the PPC substance was introduced intracutaneously in two-folds dilutions to clipped skin on the guinea pigs backs. In order to monitor cutaneous sensibility, physiological saline solution was administered to the same animal to another clipped skin area. The reference animals received a challenging dose of the substance (equal to the sensitizing dose). Then the animals were subjected to intravenous injections of 0.5 ml of 1% Evans blue solution. In 30 minutes the animals were sacrificed with ether; the size of the blue spot on the internal side of skin in place of introduction was measured. The positive response is considered when the spot diameter exceeds 6 mm, not exceeding 3 mm in reference groups. Three cases of a positive response were recorded in the group receiving the substance in the dosing ten-fold exceeding the therapeutic dose. The number of responses is indicative of the possibility of individual responses.

### Delayed-type hypersensitivity test in mice

The experiments were conducted in outbred mice weighing 18-20 g, 10 animals in each group. The total amount of the experiment animals was 30. The animals were sensibilized intracutaneously to the tailset with a single dose of the PPC emulsion in Freund's incomplete adjuvant (1:1). The introduction dose was 60 µl. The reference animals were sensibilized with Freund's complete adjuvant emulsion with Hank's solution (1:1). In 5 days, in order to detect sensibilization, 40 µl of the test medication solution in Hank's solution was administered into the mice's hindpaw pads. In 24 hours upon testing, the oedema was measured using MK-0-25 micrometer. Statistical analysis of the outcome did not reveal a significant difference in experimental and reference animals' paw thickness (P>0,05). PPNC substance introduction does not induce a delayed-type hypersensitivity response.

### Peritoneal macrophage phagocytic activity study

The experiment was conducted in white rats weighing 180-200 g. The total amount of the animals was 30, 10 in each group. Two doses of the PPC preparation were studied: therapeutic dose, a dose tenfold exceeding the therapeutic dose; the third group was the reference group. The method is based on defining the lysing reagent optical density upon phagocyte breakdown, having absorbed neutral red particles. The rats exposed to the medication were decapitated. 5 ml of 199 medium containing 20% of fetal calf serum was introduced aseptically intraperitoneally. After abdomen massage, the medium was syringed out. The cell fluid portions, taken from the entire animal group, were pooled together. The live cells concentration was adjusted to 1.5x10³ cells/ml. The cell suspension was aseptically applied to 40 mm Petri dishes, 2 ml to each. The dishes were incubated for 2 hours at 37°C. The supernatant containing loose cells was poured out and the monolayer on the plastic plate was washed with 199 medium two times. Neutral red-containing solution was applied to slightly dried dishes and kept at 37°C for 1 hour. The solution was poured out. The cells were washed with 199 medium. 3 ml of lysing reagent was applied to each dish. The cells were thoroughly treated by rotating the dish. The solution was tubed and its optical density was measured using a spectrophotometer at 540 nm wavelength.

Macrophage phagocytic activity is not reduced by the PPNC substance introduction. Significant difference between experimental and reference groups outcome was not detected.

### Mast cell degranulation test

The experiment was conducted in female rats weighing 180-200 g.

The total amount of the animals was 30. The PPC substance solution was administered intraperitoneally in therapeutic dose and in a dose tenfold exceeding the therapeutic dose; the reference group was comprised of itact animals. In order to make a mast cells suspension, the animals were decapitated, buffer was introduced into the abdomen; after the anterior abdominal wall massage the mast cells suspension was collected to centrifugal tubes. The preparations were made on specimen slides stained with 0.3% alcohol solution of neutral red. 0.03 ml of an experimental animal serum and 0.03 ml of the studied medication in 1:100 dilution were added to 0.03 ml of the mast cells suspension. In the reference group, degranulation did not exceed 5%. The preparations were covered with a coverslip, its edges were sealed with paraffin. Then the preparation was incubated for 15 min in a thermostat at 37°C. The preparations were examined with X40 magnification, counting the mast cells that have undergone degranulation and normal cells in the amount of 100. The response was considered positive if degranulation degree exceeded 20%.

Introduction of 0.01 mg/kg PPC substance does not induce mast cells degranulation, does not differ from the reference parameters and exceeds the threshold value by 5%. The tenfold dosing group response was weak positive, but these values fall within the statistic error rate.

Detection of prions. Samples were taken from 4 bottles. The test was conducted by enzyme-linked immunosorbent assay method using a 96 well polystyrene plate. PrionScreen diagnosis test system and Digiscan spectrophotometer were used. Each sample was tested in 4 repeats. Reference: K-: 8 wells, K+: 8 wells. The resulting optical densities of the reference and experimental samples complied with the test conditions.

Outcome: infectious proteins and prions were not detected in the studied PPC substance.

General conclusion. The original biologically active PPC substance acute and subchronic toxicity studies were carried out, as well as the studies of its local irritant and immunotoxic effects, allergenic properties, and prion infections presence.

The PPNC pharmcomposition acute toxicity was studied by an experiment in BALB/C mice. The PPNC was administered in three ways: subcutaneously, intraperitoneally, by mouth, and intranasally. The maximum possible dosage for mice is 0.2 ml, i.e. 10 mg/kg, 100 µl of 0.1 % solution was introduced intranasally to each meatus. No deaths or acute intoxication symptoms were recorded with the PPNC pharmcomposition taken in the said dosage in any route of administration.

Subchronic toxicity was studied in mature and separately in immature Wistar rats. The studied preparation was introduced subcutaneously in therapeutic dose of 0.01 mg/kg and a tenfold dose of 0.1 mg/kg; intravenously in 0.0025 mg/kg and x10 - 0.025 mg/kg dosing. Intranasally, by one spray to each meatus twice daily, which was 400 µl of 0.1% solution - 0.016 mg/kg was studied in chinchilla rabbits. In course of toxicity study, the standard methods of haematologic and biochemical blood analysis and visceral organs histologic examination were used.

The studies showed that the PPNC pharmcomposition administered in various routes, both in therapeutic and tenfold dosage, for one month did not result in visceral organs modification, nor did it make a toxic effect on the blood system. The pharmcomposition did not make a local irritant effect in various routes of administration.

Allergenic properties study was performed in guinea pigs with intracutaneous applications. The PPNC pharmcomposition introduced in therapeutic dose did not induce an allergic response. Two cases of animals' hypersensitiveness to the medication were recorded when introduced in the dose tenfold exceeding the therapeutic dose. Immunotoxicity study was performed in Wistar rats macrophages, which did not reveal the said effect.

The PPNC pharmcomposition study was performed for prion infections presence by enzyme-linked immunosorbent assay method. The PPNC was free from expected agents.

### Example 9. Mutagenicity of the pharmcomposition.

The following set of tests was used to assess mutagenic properties of the claimed PPNC pharmcomposition [19]:
- gene mutation account in Ames test of Salmonella / microsome using Salmonella typhimurium TA 97, TA 98 and TA 100 strains as test objects;
- micronuclei account in mice marrow cells.

A sample of protein/polypeptide pharmcomposition was tested, which was transparent liquid, bottled in a sterile dark glass bottle in the amount of 20 ml, containing the substance in 0.1 mg/ml concentration.

The protein/polypeptide/nucleotide pharmcomposition mutagenic properties assessment in Ames test.

Mutational test on Salmonella typhimurium is a bacterial test system for account of gene mutation to histidine prototrophy when subjected to chemical compounds and/or their metabolites, inducing base substitution or reading frame shift mutations in this organism genome. Mutagens inducing base pair substitution are agents inducing base pair substitution mutations in DNA molecule. Mutagens inducing genetic code reading frame shift mutations are agents inducing excess or deficiency of single or multiple base pairs in DNA molecule.

This method is used to detect the ability of pharmaceutical substances or their metabolites to induce gene mutations in Salmonella typhimurium indicator strains. The bacteria are treated with the compound being tested with metabolic activation system (CM+) or without metabolic activation (CM-). Upon incubation for a certain period, the number of revertant is counted in different test strains in comparison to the number of spontaneous revertants in negative control options (untreated cultures or solvent treated cultures). If a compound and/or its metabolites being tested are mutagenically active, they will induce back mutations from histidine auxotrophy to prototrophy in histidine-dependent Salmonella typhimurium strains.

For testing, a set of Salmonella typhimurium indicator strains was used, allowing for recording reading frame shift (TA 98 and TA 97) and base substitution (TA 100) mutations. Strains were taken from Russian National Collection of Industrial Microorganisms of Research Institute for Genetics and Selection of Industrial Microorganisms («Genetika»).

The procedure for handling bacterial cultures, strain genotype check, regulations for their museum storage, necessary experimental equipment, labware, chemical agents, nutritional media and solutions, preparation of rat liver homogenate and activating mixture, and findings statistical analysis methods complied with standards as described in reference literature.

For metabolic activation, the S9 Wistar male rats liver fraction was used; 5 days prior to sacrifice the rats were subjected to microsomal enzymes inducer introduction - sovol (300 mg/kg, single dose, intraperitoneally). In order to monitor the metabolic activation system, ethidium bromide was used (10 µg/dish) on TA 98 strain at CM+.

The PPNC pharmcomposition in form of sterile solution with equal protein and nucleotide content - 0.1 mg/ml was studied: 1.0 and 0.3 ml of original solution per dish and three tenfold dilutions in physiological solution (0.1 ml/dish). The substance test doses were 300; 100; 10; 1 and 0.1 µg/dish.

The experiment was supported with positive controls using substances inducing mutations in corresponding tester strains, with and without activation conditions. In cases without activation, sodium azide was used in the amount of 10 µg/dish for TA 100 strain at CM-; 2,7- diamino -4,9- dioxy -5,10- dioxo-4,5,9,10-tetrahydro-4,9- diazopyrene (DIAM) - µg/dish for TA 98 strain at CM-; 9-aminoacridine (9AA) - 50 µg/dish for TA 97 strain at CM-. In order to monitor the metabolic activation system, ethidium bromide was used - µg/dish on TA 98 strain at CM+. As a negative control, physiological solution was used (0.1 ml per dish).

Selective semi-enriched agar (0.7%) in test tubes was melted on boiling-water bath at 100° C and placed to a temperature-controlled water bath at +45 - +46° C.

First, to test tubes with agar 0.1 ml of sample was applied in required dilution concentration, then - 0.1 ml of bacteria suspension and 0.5 ml of microsomal activating mixture (for metabolic activation option). Then the tube content was quickly stirred and plated out to the lower minimum agar layer in Petri dish. Duration of microsomal activating mixture application and semisolid agar plating onto the dish did not exceed 10-15 seconds. The dishes were left at room temperature for 30-40 minutes and after full solidifying were placed to a thermostat at +37°C. The results were recorded after 48 hours of incubation.

Concurrently, the experiment included options without a metabolic activation system (CM-) and with a metabolic activation system (CM+). In the CM- option, direct mutagens effect can be recorded due to the original substance structure activity. Meanwhile, the effect of promutagens, i.e. compounds whose effect is related to formation of mutagenic metabolites, can be taken into account in comparing CM- and CM+ options of the substance study analysis findings. In each reference and experimental option 2 dishes were used. The mutagenic effect was considered significant if the average number of revertant colonies per dish in the experiment option twice and more exceeded that of the reference option. The experiment results were taken into account provided there was a standard response in all options of positive and negative control. The number of revertant colonies in control with solvent in options CM- and CM+ fell within the range of spontaneous level variations for these strains. The strains response to standard mutagens fell within the range of ordinary levels.

The studied PPNC pharmcomposition in all concentrations tested showed no mutagenic effect on TA 100, TA 98 and TA 97 strains in options with and without metabolic activation system.

CONCLUSION: the PPNC pharmcomposition does not induce gene mutations in Salmonella typhimurium test strains within the entire range of tested concentrations.

The PPNC pharmcomposition mutagenic properties assessment by micronucleus method in mammalian cells.

Cytogenetic activity is the ability of a substance to cause structural and numeral chromosomal distortions in somatic and germ cells.

Micronuclei are small DNA-containing formations consisting of acentric chromosome fragments or ana-telophase laggards. At telophase stage, these fragments can be incorporated into daughter cell nuclei or form single or multiple micronuclei in cytoplasm.

The study objective was detection and quantitative estimate of cytogenetic (mutagenic) activity of pharmacologic substances in polychromatophil cells (PCC) of mammal marrow. The method is based on microscopic recording of cells with micronuclei.

The experiments were performed to 2 months old male and female F1 (CBAx C57BI6/J) cross-breed mice weighing 18-20 g. Each group included 6 animals. The animals were kept at 12 hours light pattern with free food and water access. Animal experiments for assessment of mutagenic properties of the PPNC with single and five-time dosing were conducted in compliance with official records. Three sessions of experiments were carried out with corresponding controls: therapeutic dose was tested on male animals with a single introduction; subtoxic dose was tested on male animals with a single introduction; therapeutic dose was tested on male animals with a single introduction; subtoxic dose was tested on male animals and female with five-time introduction. Taking into account that the possible presumed method for usage of the PPNC pharmcomposition at a clinic was intravenous, subcutaneous, and intranasal introduction, in this study intraperitoneal introduction of the substance to mice was used. The PPNC dosing for assessment of mutagenic activity was calculated based on the maximum human recommended daily therapeutic dose (TD). Intravenous introduction of PPNC pharmcomposition was recommended in the amount of 2 ml at 0.1 mg/ml concentration. In this case, a human therapeutic dose (TD), including a child's dose, can be 0.05 mg/kg/daily. In order to calculate an experiment dosing for mice, a conversion factor is recommended that takes into account a human or animal body surface area to weight ratio. In our study, it was 8.33. Therefore, the mice TD was approximately 8.33 x human TD (0.42 mg/kg).

As a subtoxic dose, 10mg/kg, the maximum possible in this experiment conditions dosing was used, as the substance was presented in 0.1 mg/ml concentration, and the normally used amount of substance for mice is 0.1 ml per 10 g. Respectively, in conversion to humans, the maximum approved dosing is 1.7mg/kg.

Seven animal groups were formed: four experimental groups and three corresponding reference groups. The substance in the amount of 0.1 mg/kg was introduced five times at 24 hours interval to males and females. All animals were sacrificed 6 hours upon the last introduction of the protein/polypeptide complex.

In two other experimental groups, the PPNC was introduced in single dosing to males in doses of 0.2 mg/kg and 0.5 mg/kg.

Normal saline solvent was used as a negative control. It was introduced subcutaneously to male and female mice of two reference groups for the same period and in the same amount as to the experimental animals. As a positive control, 20 mg/kg cyclophosphamide was used, diluted ex tempore in normal saline at a single intraperitoneal introduction 24 hours before sacrifice.

Marrow cell preparations for micronuclei count were made by a standard method in compliance with common guidelines [20]. The animals were sacrificed through cervical dislocation 6 hours after the last introduction. Both thigh bones were detached and muscles were removed from them. In order to wash out the marrow, fetal calf serum was used (produced by NPP PanEko). The marrow from both thigh bones was washed out into a microtube using a syringe. The suspension was centrifugated at 1,000 rpm for 5 min, the supernatant was removed, and the sediment was carefully resuspended. A smear was prepared from a drop of the suspension, which was further air dried out. Then it was fixed with methanol for 3 minutes. Stained according to Romanowsky-Giemsa method. 2,000 polychromatophil cells from each animal were analyzed in encoded preparations. At counting 500 red blood cells, polychromatophilic to normochromatic erythrocytes ratio was defined. Upon analysis completion, the preparations were decoded.

The positive result criterion is the replicable and probably significant increase of micronuclei polychromatophilic erythrocytes number at least in one group compared to the reference group. Achievement of a positive result is indicative of the fact that the substance induces chromosome damage and/or cell mitotic apparatus disturbances in experimental animals.

Micronuclei count statistical analysis was performed by comparing experimental series to reference once by X2 criterion; intergroup comparison of polychromatophilic erythrocytes share was carried out using Mann-Whitney test.

Findings of micronuclei count in mice medulla polychromatophilic erythrocytes: in neither option of the experiment the PPNC pharmcomposition caused increased frequency of micronucleus cells in mice medulla when comparing experimental and corresponding reference series. Cyclophosphamide (positive control) introduced intraperitoneally to male mice in 20 mg/kg dosage caused increased frequency of micronucleus cells by 19.6 times (62.1 % against 3.16 % in reference, P<0.001). Polychromatophilic erythrocytes share in the total medulla erythrocytes was approximately on the same level in experimental and reference series groups, which is indicative of absence of the composition toxic effect on hematogenesis in tested dosing. During application of positive control (cyclophosphamide), polychromatophilic to normochromatic erythrocytes ratio was found to shift to the latter (compared to control P<0.005).

Therefore, the PPNC mutagenic activity was not detected in mice medulla polychromatophilic erythrocytes micronuclei induction test at single and five-time dosings introduced intraperitoneally to male and female mice in 0.42 mg/kg dosage, which in conversion is equivalent to a human therapeutic dose. Also, the mutagenic activity was not detected at single introduction of the protein/polypeptide pharmcomposition to males in maximum possible dosing - 10 mg/kg. The PPNC did not show toxic effect by the "polychromatophilic erythrocytes share" criterion.

### Conclusion on the PPNC mutagenic properties assessment

The PPNC does not make a mutagenic effect in Ames test of Salmonella / microsome and in mice medulla polychromatophilic erythrocytes micronuclei induction test in experiments performed in compliance with the Guidelines for Experimental (Non-Clinical) Study of New Pharmaceutical Substances.

### Example 10. The biologically active PPC substance effects on the cell population cytokinetic parameters

Materials and methods. ZTZ mice fibroblasts were cultured on DMEM medium (PanEko, Moscow) adding 584 mg/l glutamine, an antifungal and antimicrobial drug (Sigma, USA), in accordance with the manufacturer's guidelines, and 10% by volume of normalized fetal calf serum (HyClone, USA). For the experiment, the cells were plated to 24 wells plates (Corning) in concentration of 3,000 cells per ml (per well). For morphologic studies, 12 mm coverslips were placed in the wells (Ted Pella, USA), and the cells were plated onto them.

Test substances application. Upon reaching the required density by the cell culture, the culture medium was replaced with a fresh one having the same composition (control), with DMEM medium without serum with addition of 2 mg/ml of bovine serum albumin (Sigma) (negative control), with DMEM medium without serum with addition of 10% by volume of PPC (Experiment 1), and with DMEM medium with addition of 10% of serum and 10% of PPNC (Experiment 2).

Experimental procedure. The cells were analyzed 8, 24 and 48 hours after application of agents being tested. In vivo culture condition was analyzed, the cells were photoed using an Axiovert 200M phase-contrast inverted microscope (Carl Zeiss, Germany), equipped with an EC Plan-Nefluar 10x NA 0.5 lens and a digital camera ORCA II Erg-2 (Hamamatsu Photonics, Japan). For cells count, a cell sheet was dissociated with a mixture of Versene solution (PanEko) and 0.025% trypsin solution (PanEko) in 3:1 ratio. The resulting suspension was centrifugated at 300 g, the sediment was resuspended in Hank's solution (PanEko) and counted in Goryaev chamber. For cell population cytokinetic parameters analysis, the cells grown on coverslips were bound with 1% gluteraldehyde on 0.1 M phosphate buffer at +4°C, permeabilized with 0.5% Triton X-100 solution on PBS for 10 minutes, washed with distilled water, and stained with hemalum (for 90 seconds) (Sigma), dehydrated, and enclosed into DePeX. DAPI cells (0.1 µkg/ml) were also stained after permeabilization and enclosed into Moviol. The resulting preparations were examined using an Axioskop II fluorescence microscope (Carl Zeiss) with Plan-Neofluar 40x NA 1.2 and 100x NA 1.3 lenses.

### Outcome. Populations' cytokinetic parameters

*Control*. 8 hours. Mitotic index - 8%. Apoptosis and necrosis - 0%.
24 hours. Mitotic index - 11.9%. Apoptosis and necrosis - 0%. The cells count in a well is 265,000.
48 hours. Mitotic index - 9%. Apoptosis and necrosis - 0%. The cells count in a well is 780000.
Bovine serum albumin. 8 hours. Mitotic index - 5%. Apoptosis and necrosis - 0%.
24 hours. Mitotic index - 5,5%. Apoptosis and necrosis - 0%. The cells count in a well is 167500.
48 hours. Mitotic index - 3%. Apoptosis and necrosis - 0%. The cells count in a well is 625000.

### PPNC.

8 hours. Mitotic index - 6%. Apoptosis and necrosis - 0%.
24 hours. Mitotic index - 7,1%. Apoptosis and necrosis - 0%. The cells count in a well is 250000.
48 hours. Mitotic index - 6%. Apoptosis and necrosis - 0%. The cells count in a well is: calculated 187,000, actual ≈ 400,000 (emission).

### Serum + PPNC.

8 hours. Mitotic index - 8%. Apoptosis and necrosis - 0%.
24 hours. Mitotic index - 4,5% (emission). Apoptosis and necrosis - 0%. The cells count in a well is 317,500.
48 hours. Mitotic index - 9%. Apoptosis and necrosis - 1%. The cells count in a well is 680,000.

All experimental lines show biomass buildup, no cases of cytotoxic action is recorded. In the negative control, proliferation efficiency is minimum, in the PPNC presence proliferation stimulation is observed compared to the negative control. The positive control and medium with addition of the PPNC and serum show equal levels of proliferation.

Cells incubated in the PPNC presence, generate thin long tails. It is especially visible on the first day of the experiment. The effect is preserved in presence of serum on the PPNV background.

Conclusions:
1) The biologically active substance consisting of the PPC is not a cytotoxic.
2) The substance contains growth factors, but is not as efficient a proliferation stimulator as fetal blood plasma.
3) The substance induced cells shape modification with clearly visible tail generation.

### Example 11. PPNC effect on cerebral blood supply andrats survival rate under brain ischemia conditions

The study was performed in male Wistar rats weighing 180-250 g under the conditions of irreversible bilateral common carotid artery occlusion (see Ye.Gusev, V.Skvortsova, 2001). The animals were taken from the Institute of Bioorganic Chemistry of the Russian Academy of Sciences (Pushschino) 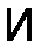 isolated for 5 days. All veterinary operations were carried out in aseptic conditions providing for preserving the experimental animals' status. The animals were kept under standard conditions [18]. In etherized rats (chemically pure diethyl ether produced by OAO Medkhimprom) a 8-10 mm section was made on the neck skin along the midline, in order to provide access to carotid arteries. Both carotid arteries were detached, separating the vagus and the sympathetic nerves, aligned with the arteries. Silk stitches were placed under the vessels (Silk blk 17 x 45 cm/M3/USP2/ Non needled/ «Ethicon Johnson & Johnson», cat.no. W203) and two people simultaneously ligatured both carotid arteries by a triple surgeon's knot. The skin incision was stitched with surgical silk (Silk blk 100cnn/M5/Sgle armed KP-3USP2/ Non needled/ «Ethicon Johnson & Johnson», cat.no. W794), putting one or two surgical cutaneous sutures and further debriding the wound with 5% iodine tincture solution. Normal saline or PPNC were administered intraperitoneally. All operations take 8-10 minutes, then the animals were caged. Three animal groups were used for the study:
- group 1 - reference animals with ligatured carotid arteries, receiving normal saline via a sterile disposable insulin syringe in the dosing of 0.5 ml per rat in one hour and 0.5 ml per rat in 5 hours upon ligation;
- group 2 - sham operated animals, which underwent the same operations as the reference animals, but without carotid arteries ligation, and received normal saline in the dosing of 0.5 ml per rat in one hour and 0.5 ml per rat in 5 hours;
- group 3 - animals with ligatured carotid arteries, receiving PPNC in the amount of 0.5 ml of 0.1 % solution (0.2 mg/kg) per rat in one hour and 0.5 ml per rat in 5 hours;
- group 4 - animals with ligatured carotid arteries, receiving PPNC in the amount of 200 µl of 0.1% solution per rat intranasally immediately prior to the operation, immediately after the etherization, in 1 and in 5 hours upon ligation.

The animals' survival rate was monitored immediately after the ligation and during the first day, i.e. the final survival rate was recorded in 24 hours. The medication biological activity showing as the brain protection under the conditions of incomplete total ischemia with simultaneous carotid arteries ligation was assessed as follows:
- high - over 80% of rats survive
- medium - 70-80% of rats survive
- low - below 70% of rats survive

For statistic data analysis, Fisher exact test with multiple comparisons was used.

The conducted experiments showed that in control experiments, upon ligation of both carotid arteries, most animals die during the first hour, i.e. 11 of 24, and another 4 animals died in one day, total 15 of 24 rats died (63%, Table 1). Among the sham operated animals, 2 rats died immediately upon the operation (17%).

Administered to rats intraperitoneally in 1 and 5 hours in the 0.2 mg/kg (0.5 ml of 0.1 % solution), the PPC caused deaths of only two animals, and 34 of 36 rats survived. Survival rate increase compared to the reference findings was 95%.

Administered to rats intranasally immediately prior to the operation and 1 and 5 hours after the operation, the PPC caused deaths of six animals, and 30 of 36 rats survived. Survival rate increase compared to the reference findings was 83%. The results are shown in Table 1.

**Table 1. The biologically active substance effect on rats survival rate under the conditions of both carotid arteries ligation**

| Animal groups | Expressed biological activity | |
|---|---|---|
| | survived | dead |
| 1 group Reference-24 rats | 9 of 24 (37%) | 15 of 24 (63%) |
| 2 group -12 rats sham operated animals | 10 of 12* (83%) | 2 of 12 (17%) |
| 3 group - 36 rats PPC 0.2 mg/kg | 34 of 36* (95%) | 2 of 36 (5%) |
| 3 group - 36 rats PPC 200 µkg, intranasally | 32 of 36* (83%) | 6 of 36 (17%) |

| | | |
|---|---|---|
| * - p ≤ 0.01 compared to reference values. | | |

Therefore, the biologically active substance, which is a protein/polypeptide/nucleotide complex, provides for a probably significant survival rate increase compared to reference values under the conditions of incomplete total brain ischemia caused by simultaneous carotid arteries ligation, both with intraperitoneal and intranasal route of administration.

### Example 12. Study of the PPNC pharmacological composition effect on the local cortex blood flow in anesthetized rats under the conditions of the total transient ischemia.

The experiments were performed in anesthetized male Wistar rats weighing 220-250 g at natural breathing. The rats were anesthetized with urethane (Sigma Aldrich Chemie GmbH, Germany) in 1,400 mg/kg dosage, intraperitoneally [21,22,23].

Methods. The animals' cerebral blood flow condition was assessed by recording the rats' local cortex blood flow using the laser Doppler flowmetry method. For registering the rats' local parietal blood flow, an ALF-21 flowmeter by Transonic System Inc. (USA) was used. For this purpose, a 0.8 mm needle sensor was fixed on the parietal region of a rat's cortex, using a micromanipulator and a yoke. Simultaneously, arteriotony variation was registered via a polyethylene cannula preliminary introduced to the femoral artery. Blood flow and arterial pressure values were registered with a BIOPAK (USA) polygraph and a PC. The PPNC pharmacological composition was administered in 0.2 mg/kg (0.5 ml of 0.1% solution) dose via a polyethylene cannula to the animals' femoral arteries.

The model of the total transient ischemia is widely used for study of both cerebral blood flow disturbances and various brain functions biochemical parameters modifications occurring after ischemic injury. The total transient ischemia in rats was caused with a 10 minutes occlusion of both common carotid arteries using special clamps with a simultaneous reduction of arterial pressure to 40-50 mm Mercury by collecting arterial blood from a polyethylene cannula preliminary introduced to the femoral artery. In 10 minutes, the clamps are removed from the carotid arteries, and the collected blood was introduced back to the animal.

The outcome was processed by analysis of variance for repeated measures and Dunnett's multiple comparison test using Biostat software.

### Findings

The experiments showed that the total transient ischemia causes a sharp decrease of local cortex blood flow in rats, at an average 6.1±1.0 c.u. or 82±2.2% of the original level. After removal of the clamps from the carotid arteries and further refusion of blood, a sharp increase of the local blood flow is observed, which gradually decreases and in 30 minutes upon reperfusion is at an average 25±2.8 c.u. or 23±2.4% of the original level (p<0.05).

The PPNC pharmacological composition introduced intravenously 30 minutes after the reperfusion in 0.2 mg/kg (0.5 ml of 0.1% solution) in as little as 10 minutes after the introduction in most experiments caused increase of local cortex blood flow in rats at an average by 12 ±3.3% (in 7 of 10 experiments). Probably significant increase of local cerebral blood flow appeared on the 50^{th} minute, was at an average 39±9.2%, and continued till the 90^{th} minute (Table 2). Here it may be noted that in reference experiments involving administration of normal saline, the level of local cerebral blood flow did not practically change.

Concurrently with the cerebral blood flow changes, arterial pressure was registered. The experiments showed that in 10 minutes upon introduction of the pharmacological substance to rats, a gradual decrease of arterial pressure was recorded, which in 50 minutes dropped at an average by 15±3.4% and remained reduced till the end of the experiment.

It should be noted that in reference experiments with normal saline introduction, a slight decrease of arterial pressure at an average by 18% is also observed; which allows to say that the PPNC pharmacological substance does not make a significant effect on arterial pressure level.

### Conclusion

Therefore, the study has shown that the PPNC pharmacological composition increases the survival rate of experimental animals under conditions of complete ligation of carotid arteries. At the same time, the pharmacological substance increases the local cortex blood flow in rats, reduced after the total transient brain ischemia, which, by the end of the experiment, exceeds the reference level recorded prior to ischemia. This allows to suggest that increase of the animals survival rate at carotid arteries ligation, affected by the pharmacological substance, can be accounted for by its ability to increase cortex blood supply in experimental animals.

Example 13. Patient L,. 22 y.o. Diagnosis: infantile cerebral palsy consequences (right hand spastic paresis with hand flexor muscle hypertonia, motor hypodynamia). The PPNC pharmcomposition was administered subcutaneously in course doses of 1 ml of 0.1% solution for 10 days at 7-10 days intervals, 3 courses 4 times a year, and intranasally 2 sprays tu each meatus (400 µl of 0.1 % solution) in a single dose in the morning for 10 days 4 courses per year. After treatment with the claimed medication for one year, combined with physiotherapeutic methods, a pronounced improvement was observed as a spastic tonus regress with a motion range increase. The patient learnt to write, peel potatoes and execute other fine movements with high accuracy. He learnt to swim. Administration of known medications during preceding 8 years was not successful.

The attached pictures 1-14 show the invented complex test and study findings.

Picture 1 shows the Example 1 protein-polypeptide complex solution UV-spectrum in the range of 200 to 500 nm wavelengths, with the absorption peak observed at wavelength of ∼(215±5) nm.

Picture 2 shows denaturing gel electrophoresis of the Example 1 protein fraction solution (2) in 5% polyacrylamide gel with Coomassie brilliant blue staining compared to the standard set of marker proteins having molecular weights ranging from 1 kDa to 250 kDa (1).

Picture 3 shows the Example 1 pharmcomposition nucleotide fraction UV-spectrum, recorded in the range of 190 to 235 nm wavelengths: the absorption peak is observed at wavelength of ∼(260±2) nm, minimum UV absorption spectrum at wavelength of (230±2) nm.

Picture 4 shows the complex nucleotide fraction electrophoresis in 1.8% agarous gel at ethydium bromide staining, compared to the standard set of markers having molecular weights ranging from 100 to 7,000 base pairs. The presence of indicative specific bands ranging from 12 to 660 kDa.

Picture 5 shows the PPNC pharmcomposition solution UV-spectrum in the range of 200 to 500 nm wavelengths with the absorption peak at wavelength of ∼(260±2) nm and minimum UV absorption spectrum at wavelength of (230±2) nm.

Picture 6 shows the Example 3 PPNC pharmcomposition (2) denaturing gel electrophoresis in 5% polyacrylamide gel compared to the standard set of marker proteins having molecular weights ranging from 1 kDa to 250 kDa (2) at argentic nitrate staining. The presence of indicative specific protein and nucleotide fraction bands is defined.

Picture 7 shows the Example 3 PPNC pharmcomposition (2) denaturing gel electrophoresis in 5% polyacrylamide gel compared to the standard set of marker proteins having molecular weights ranging from 1 kDa to 250 kDa (2) at Coomassie brilliant blue staining. The PPNC protein fraction characteristic bands are present.

Picture 8 shows the Example 2 protein-polypeptide complex solution UV-spectrum in the range of 200 to 500 nm wavelengths, with the absorption peak observed at wavelength of ∼(215±5) nm.

Picture 9 shows the Example 2 protein-polypeptide complex (2) denaturing gel electrophoresis in 5% polyacrylamide gel at Coomassie brilliant blue staining, compared to the standard set of marker proteins having molecular weights ranging from 1 kDa to 250 kDa (1).

Picture 10 shows the Example 4 pharmcomposition nucleotide fraction UV-spectrum, recorded in the range of 190 to 235 nm wavelengths: the absorption peak is observed at wavelength of ∼(260±2) nm, minimum UV absorption spectrum at wavelength of (230±2) nm.

Picture 11 shows the Example 4 pharmcomposition nucleotide fraction electrophoresis in 1.8% agarous gel at ethydium bromide staining, compared to the standard set of markers having molecular weights ranging from 100 to 7,000 base pairs. The presence of indicative specific bands ranging from 12 to 660 kDa is defined.

Picture 12 shows the Example 4 PPNC pharmcomposition solution UV-spectrum in the range of 200 to 500 nm wavelengths with the absorption peak at wavelength of ∼(260±2) nm and minimum UV absorption spectrum at wavelength of (230±2) nm;

Picture 13 shows the Example 4 PPNC pharmcomposition (2) denaturing gel electrophoresis in 5% polyacrylamide gel compared to the standard set of marker proteins having molecular weights ranging from 1 kDa to 250 kDa (1) at argentic nitrate staining. The presence of indicative specific protein and nucleotide fraction bands is defined.

Picture 14 shows the Example 4 PPNC pharmcomposition (2) denaturing gel electrophoresis in 5% polyacrylamide gel compared to the standard set of marker proteins having molecular weights ranging from 1 kDa to 250 kDa at Coomassie brilliant blue staining. The PPNC protein fraction characteristic bands are present.

The invention allows for creation of a biologically active substance with enhanced properties and high biological activity having tissue-specific reparative and regenerative action on nerve tissue, and for developing a method for producing the same by extracting biologically active protein-polypeptide complex from brain of ungulate farm embryos, which is solved by a selected set of procedures and modes. Particularly important was to determine the optimal gestation timing of fetal brain, as well as a set of reagents, their dosage, extraction and buffering regimes, to maintain effective concentration ratios of proteins, polypeptides, and nucleotides fixed evolutionarily and defining the biological activity of the complex, providing reparative and regenerative tissue-specific effect.

### Industrial Applicability

The invention is intended for use in chemical and pharmaceutical industries and medicine and is related to biologically active substance - a protein/polypeptide/nucleotide complex derived from embryonic brain tissue of hoofed farm animals, having antihypoxic, vascular, and neuroprotective effect, stimulating physiological and reparative regeneration of nerve tissue, intended for use in medicine production for central and peripheral nervous system diseases, hypoxic states, in disaster and sports medicine, the method for production of the same and a pharmcomposition based on it.

Sources of information taken into account:
1. RF Patent No.2128511 C1
2. RF Patent No.2129427 C1
3. RF Patent No.2132688 C1
4. Application of France No. 2413912
5. RF Patent No.2132688 IPC A61 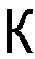 35/48
6. EA No.004788 B1
7. EA No.003301 B1
8. RF Patent No.1298979, cl. A 61 K 35/50, 1993 r
9. RF Patent No.2237486 C1
10. UA Patent No.15241, IPC 6 A 61 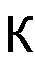 35/50, Bul. No.4, 2000
11. International Application No.WO 2009/088314 A1, 2007
12. « 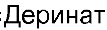 » 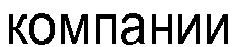 « 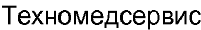 » 3AO 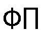 ( 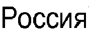 ). ( 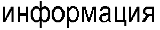 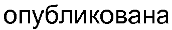 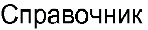 « 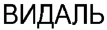 » 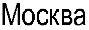 , « 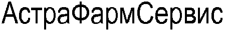 » 2010 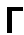 ., 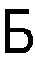 -506) (Derinat produced by ZAO Pharmaceutical Company "Technomedservis" (Russia) (the information was published in "Vidal" Drug Reference Book, Moscow, AstraPharmService, 2010, B-506)).
13. « 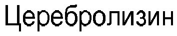 » 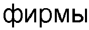 «Ebewe» ( 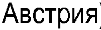 ) ( 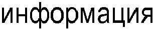 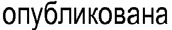 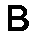 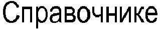 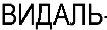 -2007, 3a 2007 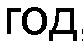 , cTp. B-1195) (Cerebrolysin produced by Ebewe (Austria) (the information was published in "Vidal" Drug Reference Book, 2007, page B-1195))
14. « 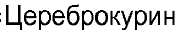 » 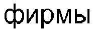 OOO « 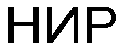 » ( 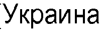 ) 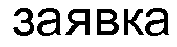 97119997/14, 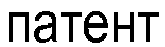 (19) RU (11) 2128511 (Cerebrocurin produced by OOO NIR (Ukraine), application 97119997/14, patent (19) RU (11) 2128511)
15. « 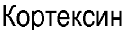 » 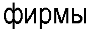 OOO « 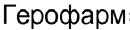 » ( 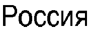 ), 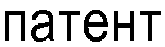 (19) RU (11)2104702 (Cortexin produced by OOO Gerofarm (Russia), patent (19) RU (11)2104702)
16. « 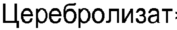 » 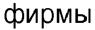 « 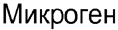 » 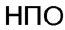 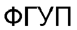 ( 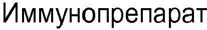 ) ( 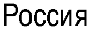 ), 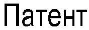 N010068 (Cerebrolysat produced by Microgen NPO FGUP (Immunopreparat) (Russia), patent N010068)
17. 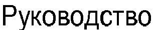 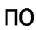 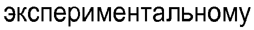 ( 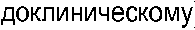 ) 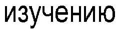 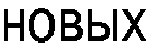 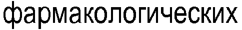 . 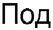 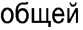 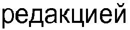 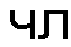 . 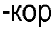 . PAMH, 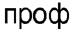 . P.Y. 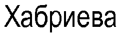 .- 2 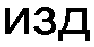 ., 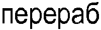 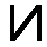 . - M, OAO 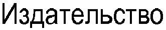 « 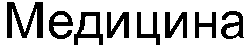 » - 2005., c.131-170 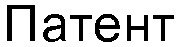 PΦ Nº 2128511(1999)/ (Guidelines for Experimental (Non-Clinical) Study of New Pharmaceutical Substances. Under the general editorship of Associate Member of RAMS professor R.U.Khabriyev. - 2nd edition, revised and enlarged - M., OAO Izdatelstvo Meditsyna - 2005, pages 131-170 RF Patent No. 2128511 (1999))
18. 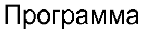 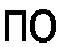 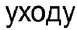 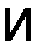 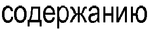 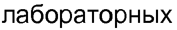 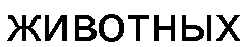 Hnn « 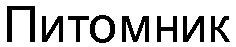 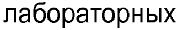 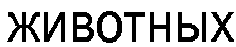 » 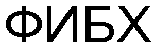 PAH oT 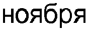 2005 r. (A Program for Care and Management of Laboratory Animals of the Nursery for laboratory animals of the Institute of Bioorganic Chemistry of the Russian Academy of Sciences, dated November, 2005)
19. 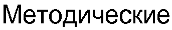 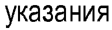 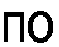 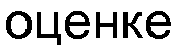 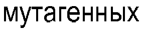 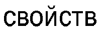 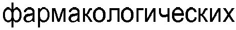 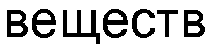 // 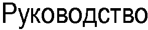 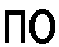 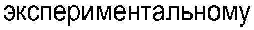 ( 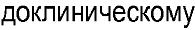 ) 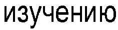 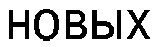 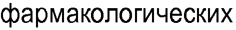 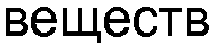 . 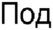 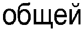 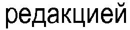 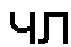 . 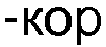 PAMH, 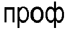 . P.Y. 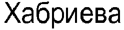 .- 2 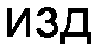 ., 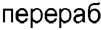 . 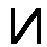 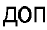 . -M, OAO 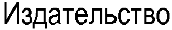 « 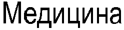 » - 2005., c.100-122. (Instructional Guidelines for Pharmaceutical Substances Mutagenic Properties Assessment // Guidelines for Experimental (Non-Clinical) Study of New Pharmaceutical Substances. Under the general editorship of Associate Member of RAMS professor R.U.Khabriyev. - 2nd edition, revised and enlarged - M., OAO Izdatelstvo Meditsyna - 2005, pages 100-122)
20. 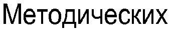 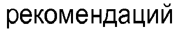 « 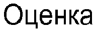 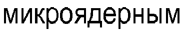 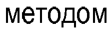 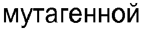 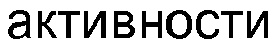 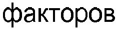 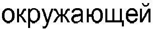 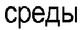 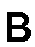 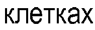 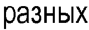 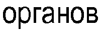 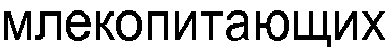 » (M., 2001). (Recommended Practice "Micronucleus Test of Environmental Factors Mutagenic Activity in Various Organs of Mammals" (M., 2001))
21. 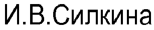 , T.C. 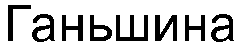 , C. 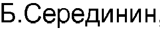 , P.C. 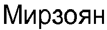 , 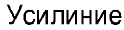 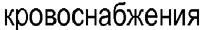 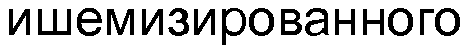 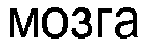 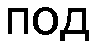 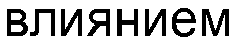 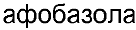 , 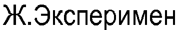 . 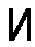 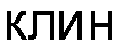 . 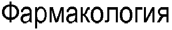 , 2004, T.67, Nº5, c.9-13. (I.Silkina, T.Ganshina, S.Seredinin, R.Mirzoyan. Increase of Ischemic Brain Blood Supply under the Stimulus of Afobazole. Journal "Eksperimentalnaya i klinicheskaya farmakologiya" ("experimental and Clinical Pharmacology'), 2004, vol.67, N.5, p.9-13)
22. Smith M.L., Bendek G., Dahlgen N. at all, Models for studing, long-term recovery following forebrain ischemia in the rat. A 2-vessell occlusion model.// Neurol.Scan., 1984, v.69, p.385 - 400.
23. Φ Y.Wang-Fisher. Manual of Stroke Models in rats. CRC press, 2008, p.3-4

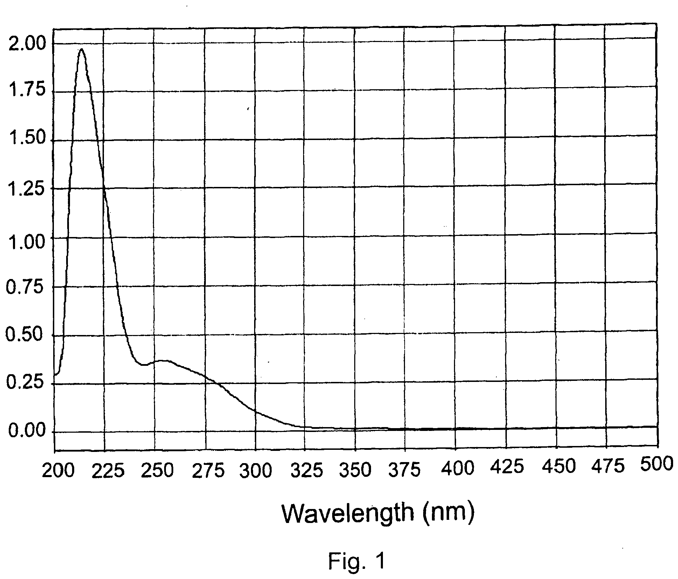

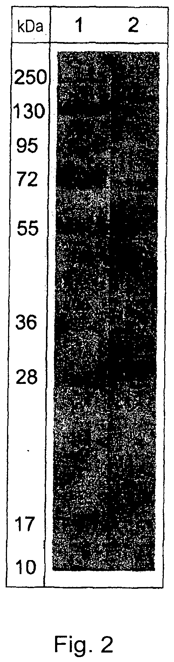

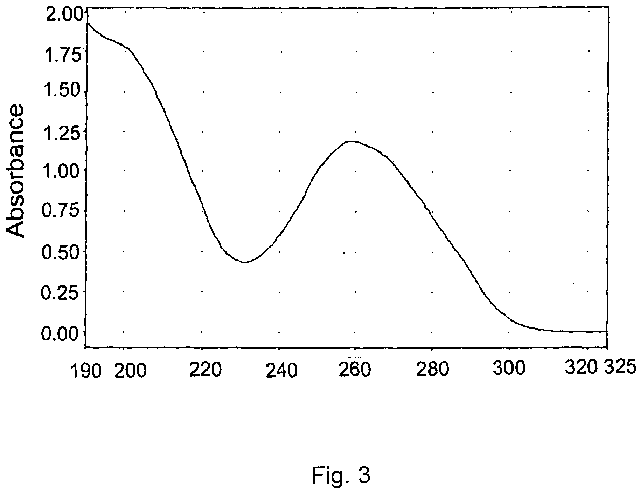

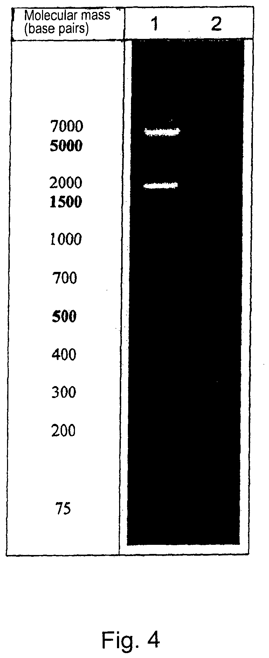

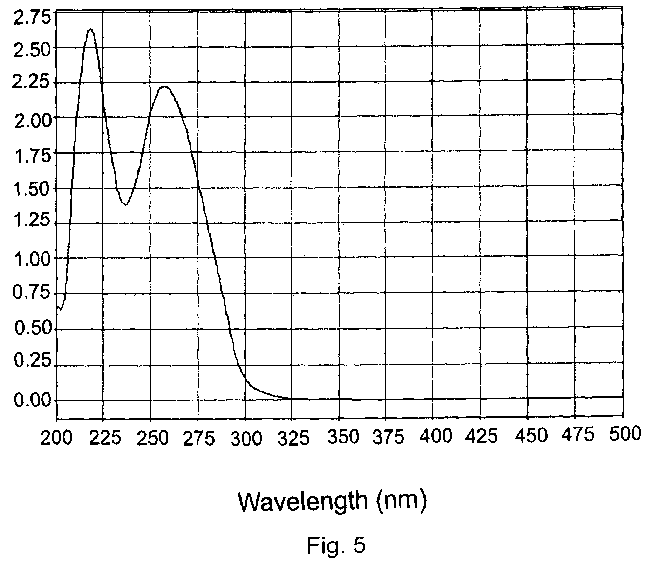

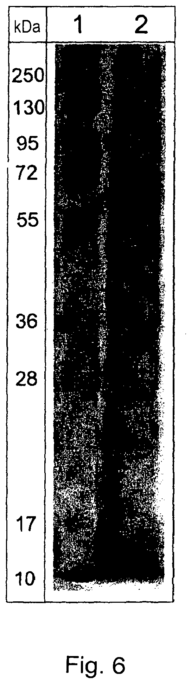

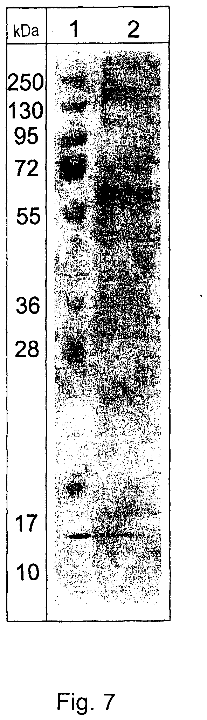

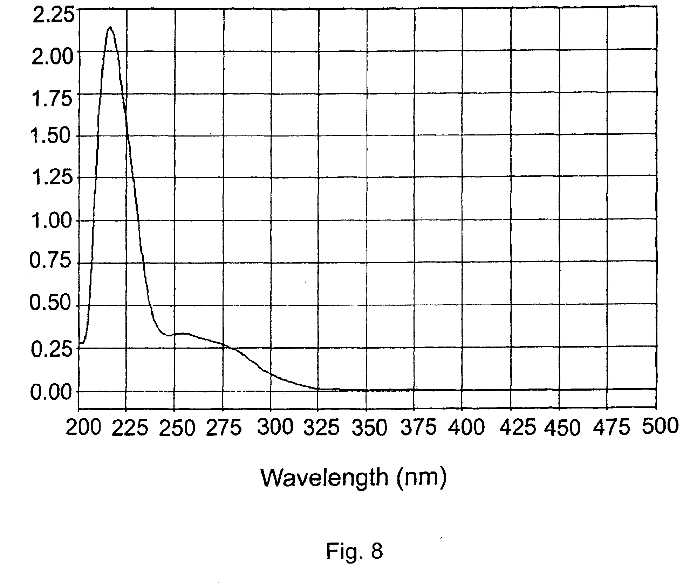

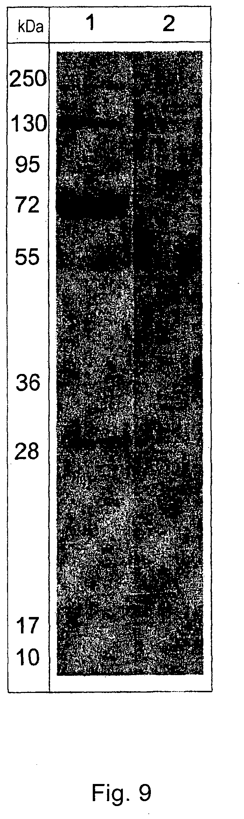

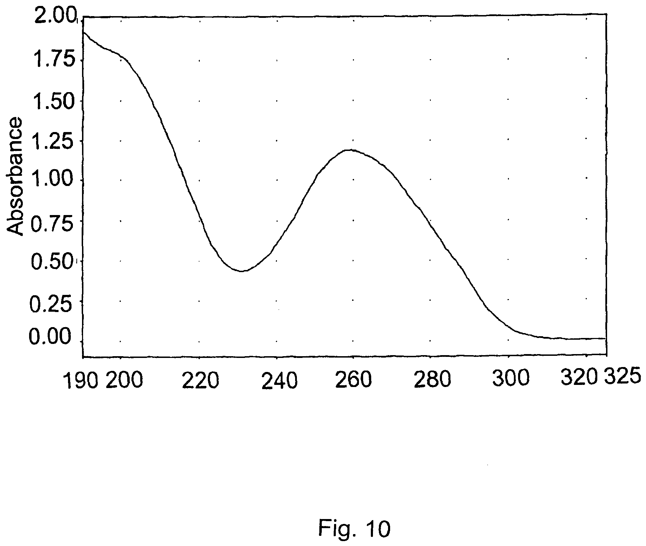

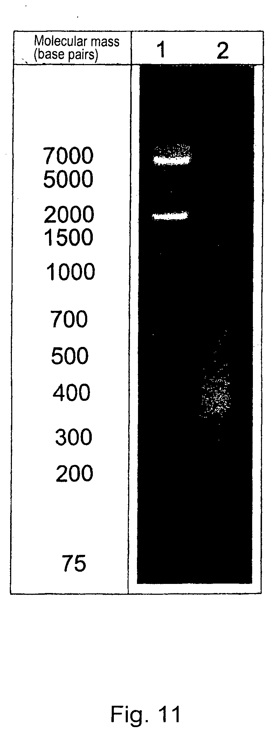

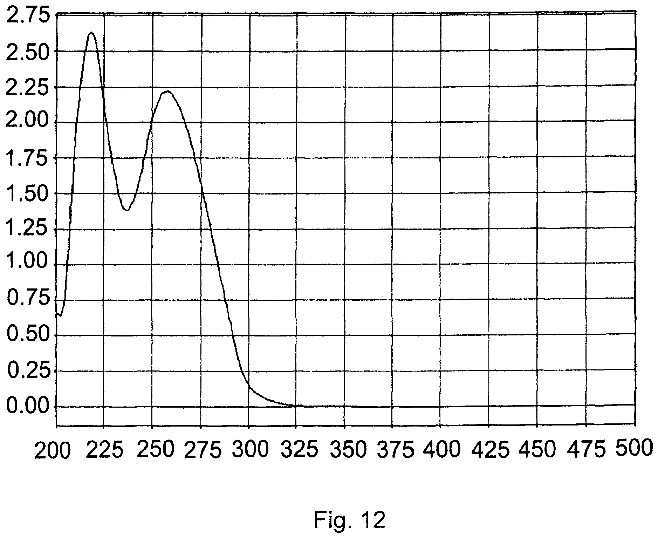

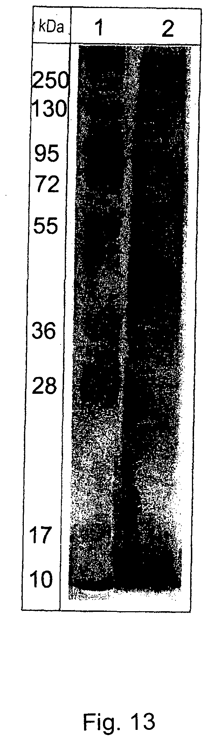

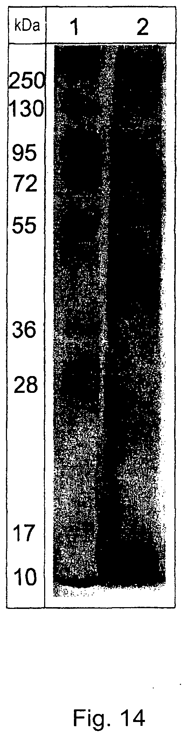

## Claims

1. Derivation method of protein-peptide complex (PPC), possessing antihypoxic, tissue-specific reparative action on the central and peripheral nervous system, consisting in the following stages performed with the nervous tissue of hoofed livestock embryos within the gestation period from the middle of the first third till the middle of the last third of pregnancy:
- homogenization in the buffer solution with simultaneous extraction in the presence of reversible proteolysis inhibitors and non-ionic detergents at pH at least 5.2 and not higher than 8.5 with further centrifugation with g value within the range of 10,000 - 28,000 during 90-30 minutes respectively;
- obtained supernatant is filtered;
- filtrate is subject to anion-exchange chromatography on the column with anion-exchange carrier, proteins and peptides connected with the carrier are separated, using for a buffer solution as a mobile phase, initially having ionic strength (I) not higher than 0.1 mmol/L, increasing it gradually or stepwise by step 0.025 mmol/L and starting collection of target fractions with the eluent ionic strength from 0.125 to 0.15 mol/L with pH from 5.2 to 8.5;
- obtained fractions are combined and subject to sterilization filtration.

2. Protein-peptide complex (PPC) possessing antihypoxic, tissue-specific reparative action on the central and peripheral nervous system, derived from the embryonic nervous tissue of hoofed livestock within the gestation period from the middle of the first third till the middle of the last third of pregnancy, by method according to claim 1, including tissue-specific negatively charged slightly acidic, neutral proteins and peptides, relative to the growth factors, differentiation, signaling molecules, determining its biological and pharmaceutical activity, with molar weights from 0.5 to 200 kDa, while at least 70% of the total protein weight has molar weight within the range of 20 - 160 kDa, possessing specific set of stripes at the SDS-polyacrylamide gel electrophoresis («SDS-Page») in 5% polyacrylamide gel, in contrast to the standard set of marking proteins with the molar weight range from 1 kDa to 250 kDa and absorption peak at the wave length 215±5 nm upon removal of UV-spectrum in the wave length range from 200 to 500 nm.

3. Pharmaceutical formulation possessing antihypoxic, immune modulating, tissue-specific reparative action on the nervous tissue, prepared in the form of solution containing biologically active protein-peptide complex as an active ingredient according to claim 2 with the content 0.05 - 2.0 mg/ml, derived by the method according to claim 1, and polynucleotide fraction, derived by alkaline hydrolysis of embryonic tissue homogenate centrifugate, left after derivation (separation) of protein-peptide fraction in the form of protein-peptide complex with further purification and precipitation of the target fraction with ethanol, derived by the method according to claim 1, in the form of DNA sodium salt, demonstrating specific immune adjusting activity combined with the protein-peptide fraction, in at least equal content with the protein-peptide complex, and pharmaceutically acceptable diluting agent.

4. Pharmaceutical formulation according to claim 3, **characterized in that** being a pharmaceutically acceptable diluting agent, it contains pharmacopeial buffer solution and, possibly, auxiliary substances including high-molecular compounds, stabilizers, preservatives and solubilizers.

5. Pharmaceutical formulation according to claim 3, **characterized in that** it contains polynucleotide fraction in the form of medium and low molecular fraction of DNA sodium salt - from 12 to 660 kDa (18 - 1000 base pairs) having maximum absorption UV-spectrum at the wave length of 260±2 nm and the minimum one - at the length of 230±2 nm in the zone of wave lengths from 190 to 325 nm with the ratio D_{260/280} from 1.6 to 2.0, presence of characteristic specific stripes upon electrophoresis in 1.8% agarose gel upon coloring with ethydium bromide, in contrast to the standard set of markers with the range of molar weights from 75 to 7000 base pairs and dark blue coloring upon qualitative reaction to ribose, and protein addition, upon Lowry content determination, not higher than 0.18%.

6. Pharmaceutical formulation according to claim 3, **characterized in that** it is adapted for parenteral application - intradermally or subcutaneously, intranasally or applicationally to mucous membranes and skin.

## Patentansprüche

1. Verfahren zur Herleitung eines Protein-Peptid-Komplexes (PPC) mit antihypoxischer Aktivität, gewebsspezifischer Reperaturaktivität auf das zentrale und periphere Nervensystem, bestehend aus den folgenden Stufen, durchgeführt mit dem Nervengewebe von Embryos von Huftieren innerhalb der Tragezeit von der Mitte des ersten Drittels bis zur Mitte des letzten Drittels der Schwangerschaft:
- Homogenisierung in der Pufferlösung mit gleichzeitiger Extraktion in Gegenwart von reversiblen Proteolyseinhibitoren und nichtionischer Detergenzien bei einem pH von mindestens 5,2 und nicht höher als 8,5, weiterhin mit Zentrifugation mit einem G-Wert innerhalb des Bereiches 10 000 - 28 000, entsprechend während 90-30 Minuten;
- erhaltener Überstand wird filtriert;
- Filtrat wird einer Anionenaustauschchromatographie auf der Säule mit Anionenaustauschträger unterzogen, Proteine und Peptide, welche mit dem Träger verbunden sind, werden unter Verwendung einer Pufferlösung als eine mobile Phase getrennt, die zu Beginn eine Ionenstärke (I) von nicht höher als 0,1 mmol/L aufweist, bei stetiger oder schrittweiser Steigerung von 0,025 mmol/L pro Schritt, und Beginn des Sammelns von Zielfraktionen mit dem Eluenten einer Ionenstärke von 0,125 bis 0,15 mol/L mit einem pH von 5,2 bis 8,5;
- erhaltene Fraktionen werden vereint und einer Sterilfiltration unterworfen.

2. Protein-Peptid-Komplex (PPC) mit antihypoxischer Aktivität, gewebsspezifischer Reperaturaktivität auf das zentrale und periphere Nervensystem, hergeleitet aus dem embryonischen Nervengewebe von Huftieren innerhalb der Tragezeit von der Mitte des ersten Drittels bis zur Mitte des letzten Drittels der Schwangerschaft durch das Verfahren nach Anspruch 1, beinhaltend gewebsspezifische, negativ geladene, leicht saure, neutrale Proteine und Peptide, bezüglich der Wachstumsfaktoren, Differenzierungsmoleküle, Signalmoleküle, die seine biologische und pharmazeutische Aktivität bestimmen, mit Molgewichten von 0,5 bis 200 kDa, wobei mindestens 70% des gesamten Proteingewichtes ein Molgewicht innerhalb des Bereiches von 20 - 160 kDa aufweist, mit einem spezifischen Satz an Banden in der SDS-Polyacrylamid-Gelelektrophorese (»SDS-Page«) in 5% Polyacrylamidgel, im Gegensatz zu dem Standardsatz von Referenzproteinen mit dem Molgewichtsbereich von 1 kDa bis 250 kDa, und einem Absorptionsmaximum bei der Wellenlänge 215±5 nm nach Entfernen des UV-Spektrums in dem Wellenlängenbereich von 200 bis 500 nm.

3. Pharmazeutische Rezeptur mit antihypoxischer Aktivität, immunmodulierender Aktivität, gewebsspezifischer Reperaturaktivität auf das Nervengewebe, hergestellt in Form einer Lösung, enthaltend einen biologisch aktiven Protein-Peptid-Komplex nach Anspruch 2 als einen aktiven Inhaltsstoff, mit dem Gehalt 0,05 - 2,0 mg/mL, hergeleitet durch das Verfahren nach Anspruch 1, und eine Polynukleotidfraktion, hergeleitet durch alkalische Hydrolyse aus einem Zentrifugat eines Homogenisates von embryonischem Gewebe, übrig nach Herleitung (Trennung) einer Protein-Peptid-Fraktion in Form eines Protein-Peptid-Komplexes, hergeleitet durch das Verfahren nach Anspruch 1 mit weiterer Aufreinigung und Ausfällen der Zielfraktion mit Ethanol, in Form eines DNA Natrium-Salzes, das kombiniert mit der Protein-Peptid-Fraktion, bei mindestens gleichem Gehalt mit dem Protein-Peptid-Komplex, spezifische immunanpassende Aktivität zeigt, und ein pharmazeutisch akzeptables Verdünnungsmittel.

4. Pharmazeutische Rezeptur nach Anspruch 3, **dadurch gekennzeichnet, dass** ein pharmazeutisch akzeptables Verdünnungsmittel ist, was Pufferlösung aus einem Arzneibuch, und, möglicherweise, Hilfsstoffe inklusive hochmolekularer Verbindungen, Stabilisatoren, Konservierungsmittel und Lösungsvermittler, enthält.

5. Pharmazeutische Rezeptur nach Anspruch 3, **dadurch gekennzeichnet, dass** sie eine Polynukleotidfraktion enthält, in Form einer mittel- und niedermolekularen Fraktion von DNA Natrium-Salz - von 12 bis 660 kDa (18 - 1000 Basenpaare) mit maximaler Absorption im UV-Spektrum bei der Wellenlänge 260±2 nm und der minimalen bei der Wellenlänge 230±2 nm, in dem Bereich der Wellenlängen von 190 bis 325 nm mit dem Verhältnis D260/280 von 1,6 bis 2,0, Anwesenheit bestimmter spezifischer Banden bei Elektrophorese in 1,8% Agarosegel nach Anfärben mit Ethidiumbromid im Gegensatz zu dem Standardsatz von Referenzen mit dem Molgewichtsbereich von 75 bis 7000 Basenpaaren, und dunkelblauem Verfärben nach qualitativer Reaktion auf Ribose und Proteinzusatz, nach Gehaltsbestimmung nach Lowry, nicht höher als 0,18%.

6. Pharmazeutische Rezeptur nach Anspruch 3, **dadurch gekennzeichnet, dass** sie auf parenterale Applikation angepasst ist - intradermal oder subkutan, intranasal oder applizierbar auf Schleimhäute und Haut.

## Revendications

1. Procédé de dérivation de complexe protéine-peptide (PPC), possédant une action réparatrice spécifique de tissu, anti-hypoxique, sur le système nerveux central et périphérique, constitué des étapes suivantes effectuées avec le tissu nerveux d'embryons d'ongulés dans la période de gestation du milieu du premier tiers jusqu'au milieu du dernier tiers de la grossesse :
- homogénéisation dans la solution tampon avec extraction simultanée en présence d'inhibiteurs de protéolyse réversibles et de détergents non ioniques à un pH d'au moins 5,2 et non supérieur à 8,5 avec en outre une centrifugation avec une valeur de g dans la plage de 10000 à 280000 durant 90 à 30 minutes respectivement ;
- le surnageant obtenu est filtré ;
- le filtrat est soumis à une chromatographie d'échange d'anions sur la colonne avec un support d'échange d'anions, les protéines et les peptides associés avec le support sont séparés, en utilisant une solution tampon comme phase mobile, ayant initialement une force ionique (I) non supérieure à 0,1 mmol/l, en l'augmentant graduellement ou par étape par étape de 0,025 mmol/l et en commençant la collecte des fractions cibles avec l'éluant de force ionique de 0,125 à 0,15 mol/l avec un pH de 5,2 à 8,5 ;
- les fractions obtenues sont combinées et soumises à une filtration de stérilisation.

2. Complexe protéine-peptide (PPC) possédant une action réparatrice spécifique de tissu, anti-hypoxique, sur le système nerveux central et périphérique, dérivé du tissu nerveux embryonnaire d'ongulés pendant la période de gestation du milieu du premier tiers jusqu'au milieu du dernier tiers de la grossesse, par le procédé selon la revendication 1, comprenant des protéines et des peptides neutres, légèrement acides chargés négativement spécifiques de tissu, relativement aux facteurs de croissance, la différenciation, aux molécules de signalisation, en déterminant son activité biologique et pharmaceutique, avec des poids molaires de 0,5 à 200 kDa, alors qu'au moins 70 % du poids de protéines total a un poids molaire dans la plage de 20 à 160 kDa, possédant un jeu de bandes en électrophorèse sur gel de SDS-polyacrylamide (SDS-Page) dans un gel à 5 % de polyacrylamide, au contraire du jeu standard de protéines marqueurs avec la plage de poids moléculaire de 1 à 250 kDa et un pic d'absorption à la longueur d'onde de 215 ± 5 nm après enlèvement du spectre UV dans la plage de longueur d'onde de 200 à 500 nm.

3. Formulation pharmaceutique possédant une action réparatrice spécifique de tissu, modulatrice de l'immunité, anti-hypoxique sur le système nerveux, préparée sous forme de solution contenant le complexe protéine-peptide biologiquement actif comme ingrédient actif selon la revendication 2 avec la teneur de 0,05 à 2,0 mg/ml, dérivé par le procédé selon la revendication 1, et une fraction de polynucléotide, dérivée par hydrolyse alcaline de centrifugeat d'homogénat de tissu embryonnaire, laissé après dérivation (séparation) de la fraction protéine-peptide sous la forme du complexe protéine-peptide avec une autre purification et une précipitation de la fraction cible avec de l'éthanol, dérivé par le procédé selon la revendication 1, sous la forme de sel de sodium d'ADN, démontrant une activité d'ajustement immunitaire spécifique combinée avec la fraction protéine-peptide, en au moins une teneur égale avec le complexe protéine-peptide, et un agent de dilution pharmaceutiquement acceptable.

4. Formulation pharmaceutique selon la revendication 3, **caractérisée en ce qu'**étant un agent de dilution pharmaceutiquement acceptable, elle contient une solution tampon de pharmacopée et, éventuellement, des substances auxiliaires, comprenant des composés de haut poids moléculaire, des stabilisants, des conservateurs et des solubilisants.

5. Formulation pharmaceutique selon la revendication 3, **caractérisée en ce qu'**elle contient une fraction de polynucléotide sous forme de fraction moléculaire médiane et faible de sel de sodium d'ADN - de 12 à 660 kDa (18 à 1000 paires de bases) ayant un spectre UV d'absorption maximum à la longueur d'onde de 260 ± 2 nm et le spectre minimum - à la longueur d'onde de 230 ± 2 nm dans la zone de longueurs d'onde de 190 à 325 nm avec le rapport D_{260/280} de 1,6 à 2,0, la présence de bandes caractéristiques spécifiques après électrophorèse dans un gel d'agarose de 1,8 % après coloration avec du bromure d'éthydium, au contraire du jeu standard de marqueurs dans la plage de poids molaires de 75 à 7000 paires de bases et une coloration bleu sombre après la réaction qualitative au ribose, et l'addition de protéines, après la détermination de la teneur de Lowry, non supérieure à 0,18 %.

6. Formulation pharmaceutique selon la revendication 3, **caractérisée en ce qu'**elle est adaptée pour l'application parentérale - intradermique ou sous-cutanée, par voie intranasale ou par application aux membranes muqueuses et à la peau.
